# EUROPEAN PATENT SPECIFICATION

(11) **EP 0 423 839 B1**
(45) Date of publication and mention of the grant of the patent: **16.08.2001**
(21) Application number: 90121584.8
(22) Date of filing: 10.12.1986
(51) Int. Cl.: C07H 21/00

(54) **Polynucleotides and probes useful in solution phase nucleic acid sandwich assay**
Polynukleotide und Proben mit Verwendung in Nukleinsäure-Sandwichassay in Lösungsphase
Polynucléotides et sondes utiles dans les essais de l'acide nucléique en phase solution

(30) Priority: 11.12.1985 US 807624
(43) Date of publication of application: 24.04.1991
(62) Divisional of application: 86309622.8
(73) Proprietor: Bayer Corporation, East Walpole, MA 02032 (US)
(72) Inventor: Urdea, Michael Steven, Alamo, California 94507 (US); Warner, Brian, Martinez, California 94553 (US); Horn, Thomas, Berkeley, California 94708 (US)
(74) Representative: Bizley, Richard Edward

(56) References cited:
- EP-A- 0 063 879
- EP-A- 0 151 001
- EP-A- 0 212 951
- WO-A-86/02929
- WO-A-86/06726
- BIOCHEMISTRY, vol. 19, no. 9, 29th April 1980, pages 1774-1781, American Chemical Society; D.E. DRAPER et al.: "A method for linking fluorescent labels to polynucleotides: Application to studies of ribosome-ribonucleic acid interactions"
- RADIOCHEM. RADIOANAL. LETTERS, vol. 35, nos. 4-5, 1978, pages 211-214; B. CERNY et al.: "Preparation of some tritium labelled nucleosides by catalyzed exchange in solution"
- NUCLEIC ACIDS RESEARCH, vol. 9, no. 5, 1981, pages 1203-1217, IRL Press Ltd, L.H. SCHULMAN et al.: "Attachment of protein affinity-labeling reagents of variable length and amino acid specificity to E. coli RNA fMet"
- JOURNAL OF ORGANIC CHEMISTRY, vol. 44, no. 18, 31st August 1979, pages 3203-3207, American Chemical Society; R.E. ROYER et al.: "Reactivity-selectivity properties of reactions of carcinogenic electrophiles with biomolecules. Kinetics and products of the reaction of benzo[a]pyrenyl-6-methyl cation with nucleosides and deoxynucleosides"

## Description

This invention relates generally to polynucleotides and probes useful in solution phase nucleic acid sandwich assays. The invention specifically relates to labeled, modified nucleotides which are incorporated in the probes.

Meinkoth and Wahl, Anal. Biochem., (1984) 138:267-284, provide a review article of hybridization techniques. See also Leary et al., Proc. Natl. Acad. Sci. USA (1983) 80:4045-4049, for a description of the dot blot assay. Sandwich hybridization is described by Ranki et al., Curr. Top. Microbiol.- Immunology (1983) pp. 308ff. See also Ranki et al., Gene (1983) 21:77-85, Virtanen et al., Lancet (1983) 381-383, and U.S. Patent No. 4,486,539. EPA 123,300 describes biotin-avidin complexes for use in detecting nucleic acid sequences. Sung, in Nucl. Acids Res. 9(22):6139-6151 (1981) and in J. Org. Chem. 47:3623-3628 (1982), discusses the synthesis of a modified nucleotide and application of the modified structure in oligonucleotide synthesis. Modified nucleotides are also discussed in Draper, Nucleic Acids Res. 12:2:989-1002 (1984), wherein it is suggested that cytidine residues in RNA be modified so as to bind to reporter molecules. Later work suggests similar modification of cytidine residues in DNA (Anal. Chem. 157(2):199 (1986). European Patent Application 063879, filed 6 April 1982, and PCT Application No. PCT/US84/00279 also describe modified nucleotides and applications thereof.

The increasing ease of cloning and synthesizing DNA sequences has greatly expanded opportunities for detecting particular nucleic acid sequences of interest. No longer must one rely on the use of immunocomplexes for the detection of pathogens, lesions, antigens, and the like. Rather than detecting particular determinant sites, one can detect DNA sequences or RNA sequences associated with a particular cell. In this manner, diseases can be diagnosed, phenotypes and genotypes can be analyzed, as well as polymorphisms, relationships between cells, and the like.

For the most part, analyses of DNA sequences have involved the binding of a sequence to a solid support and hybridization of a complementary sequence to the bound sequence. The annealing and complexing step usually involves an extended period of time and requires careful washing to minimize non-specific background signals. There is substantial interest in developing new techniques for analyzing nucleic acid sequences, which are more rapid, minimize the number of manipulative steps, and provide for an increased signal to noise ratio.

This invention is conerned with polynucleotide probes useful in such techniques. The majority of polynucleotide probes in current use are radioactively labeled, e.g. with isotopes of hydrogen (³H), phosphorus (³²P), carbon (¹⁴C) or iodine (¹²⁵I). These materials are relatively simple to synthesize by direct inclusion of the radioactive moieties, e.g. by kinasing with ³²P-labeled ATP, equilibrating with tritiated water, or the like. As is well known, however, use of such radioactive labels has drawbacks, and other detectable species which are not radioactive are preferred.

In order to incorporate other, non-radioactive types of detectable species in a nucleotide, some sort of chemical modification of the nucleotide is required. It is widely recognized that nucleotide modification is a difficult and sensitive procedure, as any modification reaction has to be mild enough to leave the RNA or DNA molecules intact, while giving a modified nucleotide product which can participate in normal base pairing and stacking interactions. These considerations typically limit nucleotide substitution positions to the 5-position of a pyrimidine and the 8-position of a purine, as noted in the literature (see, e.g., European Patent Application 063879, cited supra).

Other considerations must also be taken into account. Base pairing may be hindered during hybridization if the detectable label is at one end of the nucleotide chain rather than present at some point within it. Further, it has proved difficult to provide even non-radioactively labeled probes which may be inexpensively synthesized in large quantity. Thus, many known probes are limited in their potential applications. In copending European Patent Application No 86 309622.8, from which this application is divided, methods and compositions are described for detecting particular nucleic acid sequences. Two sets of reagents are employed, which are referred to as the capturing set and the labeling set. Each set has at least two members. The labeling set has (1) a first probe set, which comprises one or a group of first analyte complementary sequence-first label reagent recognition sequence conjugate(s); and (2) one or a group of sequences complementary to said first recognition sequence-label conjugate(s). The capturing set has (1) a second probe set, which comprises one or a group of second analyte complementary sequence(s) joined to second capturing reagent polynucleotide recognition sequence(s); (2) one or a group of sequences complementary to said second capturing recognition sequence(s) bound to a separation member or preferably a first specific binding pair member to define the capturing conjugate; and (3) a separation member joined to a first complementary specific binding pair member when (2) does not have the separation member.

The single stranded nucleic acid sample may be joined with the probes containing the complementary sequences of the two sets under annealing conditions, followed by the addition of the capturing and optionally the labeling conjugates to provide for the analyte complex with the specific binding pair member and optionally the label. The probe hybridized analyte sequence is separated by combining the complex with the separating means and separating probe bound analyte from unbound analyte. Where the label has not been previously added, the first recognition sequence-label conjugate is added to the phase containing the separation member under hybridizing conditions. The label may then be detected in either phase.

In one aspect the present invention provides a modified nucleotide given by the structure 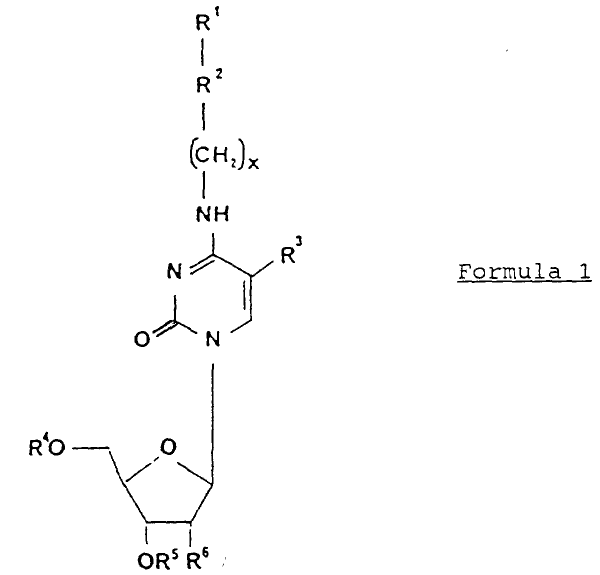 wherein R¹ is a reactive group derivatizable with a detectable label, R² is a linking moiety selected from: 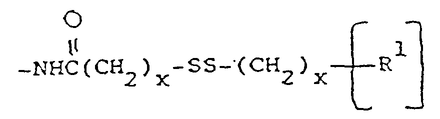 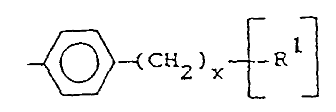 wherein x is an integer in the range of 1 to 8 inclusive, R³ is selected from the group consisting of hydrogen, methyl, bromine, fluorine and iodine, R⁴ is selected from the group consisting of hydrogen, an acid-sensitive, base-stable blocking group or an acyl capping group, R⁵ is hydrogen or a phosphorus derivative, R⁶ is H, OH, or OR where R is an acid-sensitive, base-stable protecting group, x is an integer in the range of 1 to 8 inclusive.

In another aspect the present invention provides a method of making the above modified nucleotide comprising the steps of:
reacting a pyrimidine nucleotide having the structure: 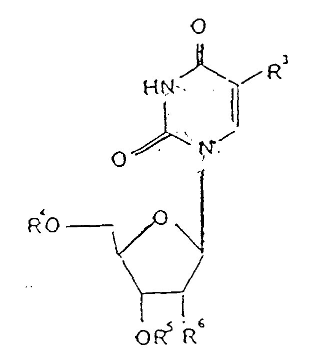 where said nucleotide is thymine, unsubstituted uracil, or uracil substituted at the 5-position with R³, wherein R³ is selected from the group consisting of hydrogen, methyl, fluorine, bromine and iodine, R⁴ and R⁵ are hydrogen, and R⁶ is hydrogen, hydroxyl, or blocked hydroxyl, with at least one protecting compound, thereby producing a 3' - and 5'- protected nucleotide: reacting said protected nucleotide with an activating agent, thereby providing an amine moiety on said nucleotide;
reacting the amine moiety of said nucleotide with a linking agent, thereby providing a structure having extending from the 4-position of said pyrimidine a linking moiety selected from 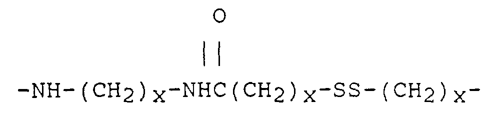 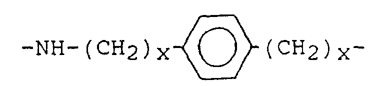 wherein X is an integer in the range of 1 to 8 inclusive and further having a free amine, carboxylic acid, or sulfhydryl reactive moiety;
reacting said free amine, carboxylic acid, or sulfhydryl moiety with a caproic acid reagent selected from the group consisting of caproic acid, an activated caproic acid ester, 6-aminocaproic acid and combinations thereof.

In making the above modified nucleotide the R¹ moiety may be derivatized with a detectable label.

In a further aspect the present invention provides polynucleotide probes having at least two nucleotides, at least one of which is given by the structure. 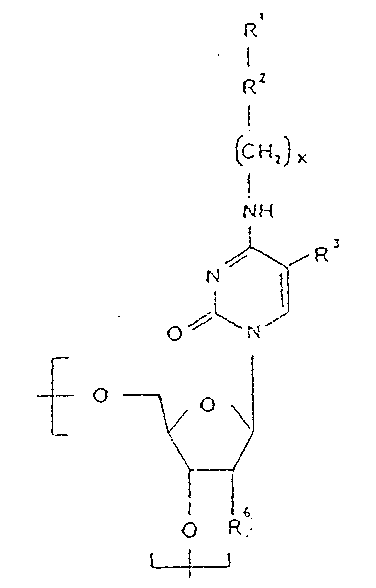 wherein R¹ is a reactive group derivatized with a detectable label, R² is a linking moiety selected from 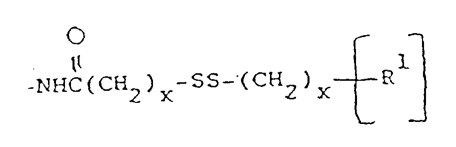 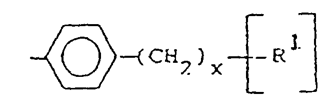 wherein x is an integer in the range of 1 to 8 inclusive, R³ is selected from the group consisting of hydrogen, methyl, bromine, fluorine and iodine, R⁶ is H, OH, or OR where R is a protecting group and x is an integer in the range of 1 to 8 inclusive.

In yet another aspect the invention provides a method of detecting a nucleotide sequence in a sample containing single-stranded or double-stranded DNA or RNA, comprising the steps of:
providing an analyte having a nucleotide sequence:
providing a labelled polynucleotide probe having a nucleotide sequence complementary to said analyte sequence, said polynucleotide probe including at least one nucleotide having the structure: 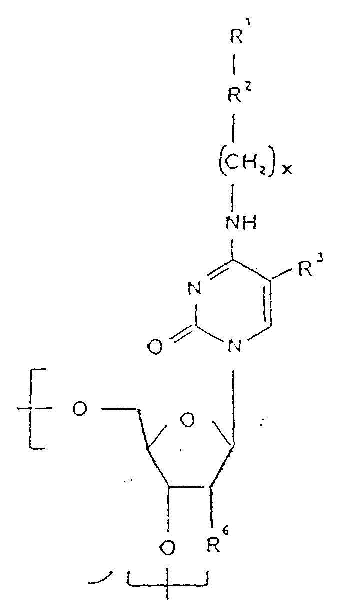 wherein R¹ is a reactive group derivatized with a detectable label, R² is a linking moiety selected from 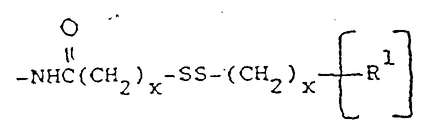 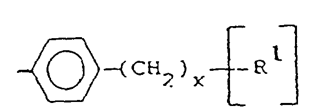 wherein x is an integer in the range of 1 to 8 inclusive, R³ is selected from the group consisting of hydrogen, methyl, bromine, fluorine and iodine, R⁶ is H, OH or OR where R is a protecting group and x is an integer in the range of 1 to 8 inclusive:
contacting said analyte with said labelled probe for a time sufficient for nucleic acid complexes to form: and
detecting the presence of any such nucleic acid complexes. In this method the labelled polynucleotide probe may be bound to a solid support. In this way the probes of the present invention can be used to screen a sample containing a plurality of single-stranded or double-stranded polynucleotide chains and will label the desired sequence if present by hybridization.

In the accompanying drawings:

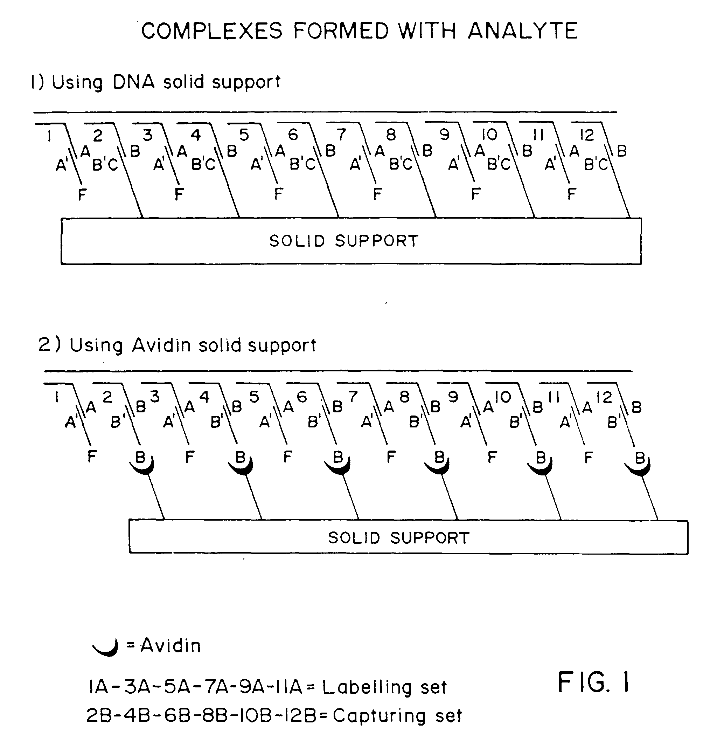

Figure 1 is an illustrative depiction of a complex from the various components bound to a solid support (1) using DNA bridges for non-covalent binding and (2) using biotin-avidin bridges for non-covalent binding.

Although not the subject of the present invention, sandwich assay methodology in which the present probes may be employed will now be described for sake of completeness.

### 1. Sandwich Assay Method

Two sets of reagents are employed. By using combinations of nucleic acid sequences complementary to a nucleic acid analyte and to arbitrary sequences and specific binding pair members, a detectable label may be separated into two phases in proportion to the amount of analyte present in a sample. By providing for annealing of nucleic acid sequences in solution, the time for performing the assay can be substantially diminished as compared to annealing on a solid surface and the number of separations and washing steps required can be limited and be less critical, so as to reduce technician error. Reagents containing complementary sequences can be added in excess during or at the end of the denaturation to inhibit renaturation of double stranded DNA and to react rapidly with the analyte strand by diffusion in solution. The rate of binding to the solid support can also be accelerated by the presence of a large amount of the binding pair member bound to the support. In addition, by adding the label conjugate as the last reagent, the analyte will be present in a highly concentrated form.

As indicated above, the method involves two sets of reagents. The first set results in labeling the analyte sequence. The second set provides the means for separating label bound to analyte from unbound label in the assay medium.

The first set, the labeling set, will involve at least two reagents and may involve 10 to 30 reagents or more. The first reagent will be a subset of nucleic acid reagents and each member of the subset will have two nucleic acid regions. The first nucleic acid region of each member of the subset will be a region complementary to a sequence of the analyte. The second nucleotide sequence will be a recognition site for the labeling reagent. This second sequence will be selected, so as not to be encountered by endogenous sequences in the sample.

The subsets will have regions complementary to the analyte sequence of at least 15 nucleotides (nt), usually at least 25nt, more usually at least 50nt, and not more than about 5kb, usually not more than about lkb. preferably not more than about 100nt. The sequence complementary to the analyte may be joined to a non-specific sequence at either or both the 5' and 3'-termini. The non-complementary sequence, if judiciously selected so as not to bind to sequences in the assay which could result in false positives, can be of any length, usually fewer than 10kb, more usually fewer than 5kb.

The complementary sequences will be chosen so as to leave areas for binding of the other reagents to the analyte. Usually, areas of at least 25nt will be left available, where the analyte sequences complementary to the sequences of the individual members of the reagent subset may be substantially contiguous or separated and members of one subset may alternate with members of the other subset. The particular pattern of binding between the two subsets may vary widely depending on the sequences of the analyte.

The reagent sequences may be prepared by synthesis in accordance with conventional procedures or by cloning and may be modified as appropriate for labeling.

The set of sequences which are complementary to the analyte may be selected based on a variety of considerations. Depending upon the nature of the analyte, one may be interested in a consensus sequence, a sequence associated with polymorphisms, a particular phenotype or genotype, a particular strain, or the like. Thus, the labeling complementary sequences will be chosen in conjunction with the other complementary sequences of the capturing set to provide information concerning the analyte.

The labeled sequence will include a sequence complementary to the first recognition sequence of the labeling probe(s). The labeling sequence will include one or more molecules, which directly or indirectly provide for a detectable signal. The labels may be bound to individual members of the complementary sequence or may be present as a terminal member or terminal tail having a plurality of labels. Various means for providing labels bound to the sequence have been reported in the literature. See, for example, Leary et al., Proc. Natl. Acad. Sci. USA (1983) 80:4045; Renz and Kurz, Nucl. Acids Res. (1984) 12:3435; Richardson and Gumport, Nucl. Acids Res. (1983) 11:6167; Smith et al., Nucl. Acids Res. (1985) 13:2399; Meinkoth and Wahl, Anal. Biochem. (1984) 138:267. The labels may be bound either covalently or non-covalently to the complementary sequence.

Labels which may be employed include radionuclides, fluorescers, chemiluminescers, dyes, enzymes, enzyme substrates, enzyme cofactors, enzyme inhibitors, enzyme subunits, metal ions, and the like. Illustrative specific labels include fluorescein. rhodamine, Texas red, phycoerythrin, umbelliferone, luminol, NADPH, α-β-galactosidase, horseradish peroxidase, etc.

The labeled sequence can be conveniently prepared by synthesis. By providing for a terminal group which has a convenient functionality, various labels may be joined through the functionality. Thus, one can provide for a carboxy, thiol, amine, hydrazine or other functionality to which the various labels may be joined without detrimentally affecting duplex formation with the sequence. As already indicated, one can have a molecule with a plurality of labels joined to the sequence complementary to the labeling sequence. Alternatively, one may have a ligand bound to the labeling sequence and use a labeled receptor for binding to the ligand to provide the labeled analyte complex.

The second set of reagents provides the means for separation of label bound to analyte from unbound label. The means for the separation or capturing means involves at least one capturing probe, usually a plurality of probes defining a subset, which includes two polynucleotide sequence regions that include a second subset of sequences complementary to the analyte, differing from the first subset of complementary sequences of the labeling probe and a recognition sequence, different from the first subset recognition sequence of the labeling probe. The second set of recognition sites for the capture probes may lie between the first set of recognition sites for the labeling probes as described above. The capturing sequences will be selected and synthesized in the same manner as described above using the considerations directing the selection for the labeling probes. Thus, the same constraints will be involved in preparing the capturing probes.

While the separating means may be directly bound to a sequence complementary to the capturing recognition sequence, preferably a specific binding pair member will be bound to the complementary sequence. The specific binding pair member will be a ligand or receptor, preferably a ligand. Ligands may be any molecules for which a naturally occurring receptor exists or can be prepared. Thus, naturally occurring ligands may be exemplified by biotin, thyroxine, enzyme substrates, steroids, and the like. Instead of naturally occurring ligands, any hapten may be employed for the production of antibodies. Ligands will generally be at least about 125 molecular weight and usually less than about 5,000 molecular weight, more usually less than about 2,000 molecular weight, and preferably less than about 1,000 molecular weight.

The receptors will generally be protein molecules and may include antibodies, naturally occurring proteins, such as avidin, thyroxine binding globulin, etc., lectins, enzymes, and the like. The receptors will generally be at least about 10,000 molecular weight, more usually 12,000 or more molecular weight, usually less than about one million molecular weight.

The specific binding pair member may be joined to the second recognition sequence by any convenient means. As already indicated, the sequence may be synthesized, providing for a convenient functionality at the terminal base, which may then be used as the linkage site. One or a plurality of specific binding pair members may be joined to the complementary sequence, depending upon the particular choice of the specific binding pair member, its size, and the nature of the functionalities. Alternatively, for a large specific binding pair member, a plurality of sequences may be joined to the binding pair member. The capturing conjugate will be prepared, so that there will be little interference, if any, from the specific binding pair member with the annealing of the complementary recognition sequences and from duplex formation with the ligand-receptor binding.

Alternatively, the receptor may be an additional nucleotide sequence that specifically recognizes the recognition sequence of the capturing probe.

The separation means can be any support which allows for a rapid and clean separation of label bound to analyte from unbound label. Thus, the separation means may be particles, a solid wall surface of any of a variety of containers, e.g., centrifugal tubes, columns, microtiter plate wells, filters, tubing, etc. Preferably, particles will be employed of a size in the range of about 0.4 to 200µ, more usually from about 0.8 to 4.0µ. The particles may be any convenient material, such as latex, glass, etc.

The homologous nucleic acid sequences need not have perfect complementarity to provide homoduplexes. In many situations, heteroduplexes will suffice where fewer than 15%, usually fewer than 10% of the bases are mismatches, ignoring loops of five or more members.

Samples of analyte nucleic acids may be from a variety of sources, e.g., biological fluids or solids, food stuffs, environmental materials, etc., and may be prepared for the hybridization analysis by a variety of means, e.g., proteinase K/SDS, chaotropic salts, etc. Also, it may be of advantage to decrease the average size of the analyte nucleic acids by enzymatic, physical or chemical means, e.g., restriction enzymes, sonication, chemical degradation (e.g., metal ions), etc. The fragments may he as small as O.1kb, usually being at least about 0.5kb and may be 1kb or higher.

In carrying out the method, the analyte sequence will be provided in single stranded form. Where the sequence is naturally present in single stranded form, denaturation will not be required. However, where the sequence is present in double stranded form, the sequence will be denatured. Denaturation can be carried out by various techniques, such as alkali, generally from about 0.05 to 0.2M hydroxide, formamide, detergents, heat, or combinations thereof. Denaturation can be carried out in the presence of the labeling probe and/or the capturing probe, so that upon change of conditions to annealing conditions, the probes will bind to any complementary sequences which are present. For example, where heat and alkali are employed, by neutralization and cooling, annealing will occur.

In many situations, it will be preferable to avoid having either the label or the separation means present during denaturation. The elevated temperatures, the non-aqueous solvents, the salts, or other materials present during denaturation may result in degradation, or undesirable modification of the label and/or separation means. Therefore, in many situations, denaturation may occur in the presence of the probes, whereupon cooling rapid annealing of the probes to the single-stranded DNA may occur, followed by the addition of the other reagents at lower temperatures and, as appropriate, under milder conditions, such as neutral pH, reduced ionic strength, or the like.

Normally, the ratio of probe to anticipated moles of analyte will be at least 1:1, preferably at least about 1.5:1, and more preferably 2:1 and may be as high as 100:1 or higher. Concentrations of each of the probes will generally range from about 10⁻⁹ to 10⁻⁶M, with sample nucleic acid concentrations varying from 10⁻²¹ to 10⁻¹²M.

After annealing conditions have been achieved, or even prior to such time, the labeled first recognition sequence and the capturing second recognition sequence are added and allowed to hybridize. Alternatively, the labeled first recognition sequence can be added after capture and separation.

A preferred embodiment which greatly reduces background and provides for extraordinarily high sensitivity will employ the following sequence. With double-stranded analyte, the analyte will be denatured in the presence of the probe or complementary sequences, or the probes may be added shortly after denaturation, and under annealing conditions. After sufficient time for annealing, the complexes may then be combined with the separation means, whereby the complexes will be bound to the support. Any background DNA or non-specifically bound DNA may be washed away so as to avoid non-specific binding of label in the next step. The solid support may then be washed to remove any non-specifically bound label to provide for a substantially reduced background of non-specifically bound label.

Consider Figure 1, part 2. In effect, the analyte which is the long bar at the top is combined with the A and B probes, where A provides the complementary sequence for the label conjugate and B provides the complementary sequence for the specific binding pair member, in this case, biotin. Thus, the A and B probes and the analyte would be joined together under annealing conditions, whereby complex formation would occur between the probes and the analyte. The biotin conjugate, B' could be included with the probes or be added in a separate step to the solution containing the analyte complexes. After sufficient time for B' to anneal to B, the resulting biotinylated analyte complex would then be added to the solid support to which avidin is bound. After sufficient time for the specific binding pair members to form complexes, the solid support could be washed free of any non-specific DNA, followed by the addition of the labeled sequence, which in this case is indicated as being fluorescein bound to A'. The labeled sequence would be added under annealing conditions and after sufficient time for duplex formation, non-specifically bound and excess labeled conjugate would be washed away and the fluorescence of the surface determined.

A somewhat shorter protocol is provided by the configuration depicted in part 1 of Figure 1. In this situation, the probes A and B would be added to the analyte under annealing conditions, whereby analyte complexes would form. After sufficient time for analyte complexes to form, the analyte complex solution would then be added to the solid support for sufficient time for the capturing probes to bind to the solid support by complex formation with the sequence indicated as B'C. Excess DNA could be washed away, followed by the addition of the fluorescein labeled sequence A', and the mixture allowed to anneal for sufficient time for complex formation to occur between the label and the probes. Excess in non-specifically bound label could then be washed away to provide the configuration depicted in Figure 1, part 1.

Usually, the denaturing step will take from about 5 to 25 minutes, usually from about 5 to 15 minutes, while the annealing step will generally take from about 30 minutes to 2 hours, frequently being completed in about 1 hour. Annealing can be carried out at a mildly elevated temperature, generally in the range from about 20°C to 50°C, more usually from about 25°C to 40°C, particularly 37°C.

Usually, an aqueous medium is employed, particularly a buffered aqueous medium, which may include various additives. Additives which may be employed include low concentrations of detergent (0.1 to 1%, salts, e.g., sodium citrate (0.017 to 0.170M), Ficoll, polyvinylpyrrolidone, carrier nucleic acids, carrier proteins, etc. Depending upon the nature of the specific binding pair members, various solvents may be added to the aqueous medium, such as dimethylform amide, dimethylsulfoxide, and formamide. These other solvents will be present in amounts ranging from 2 to 50%.

The stringency of the annealing medium may be controlled by temperature, salt concentration, solvent system, and the like. Thus, depending upon the length and nature of the sequence of interest, the stringency will be varied.

For the separation step, for example, using a ligand-receptor pair, the medium may be changed to optimize or approximately optimize the conditions for specific binding pair complex formation. Thus, the pH will usually be modified to be in the range of about 6 to 9, preferably about 7. This can be readily achieved, by adding from about 0.5 to 2, usually about 1 volume of about a 0.1 to 0.5M buffered medium, e.g., phosphate buffered saline, to the annealing medium. This medium may be added in conjunction with the separation means and the mixture allowed to incubate for at least 5min., usually about 10min., and less than about 60min., usually about 15 to 45min., more usually about 30min. being satisfactory.

The phases may then be separated in accordance with the nature of the separation means. For particles, centrifugation or filtration will provide for separation of the particles, discarding the supernatant or isolating the supernatant. Where the particles are assayed, the particles will be washed thoroughly, usually from one to five times, with an appropriate buffered medium, e.g., PBS. When the separation means is a wall or support, the supernatant may be isolated or discarded and the wall washed in the same manner as indicated for the particles.

Depending upon the nature of the label, various techniques can be employed for detecting the presence of the label. For fluorescers, a large number of different fluorometers are available. With enzymes, either a fluorescent or a colored product can be provided and determined fluorometrically, spectrophoto metrically or visually. The various labels which have been employed in immunoassays and the techniques applicable to immunoassays can be employed with the subject assays.

### 2. Nucleic Acid Probes

Nucleic acid probes useful in conjunction with the above assay method are probes which are prepared from one or more modified nucleotides. As used herein, the following definitions apply:

"Derivatizable" nucleotides are nucleotides modified so as to include at the 4-position of a pyrimidine a functional group which can react with a detectable label. An example of a derivatizable nucleotide is one which has been modified at the 4-position with an alkylamine moiety so that a free amine group is present on the structure.

"Derivatized" nucleotides are nucleotides in which the derivatizable functional group at the 4-position of the pyrimidine is bound, covalently or otherwise, directly or indirectly, to a detectable label.

"Alkylamine nucleotides" are nucleotides having an alkylamine group at the 4-position of a pyrimidine, bound to the structure in such a way as to provide a free amine group at that position.

A "polynucleotide" is a nucleotide chain structure containing at least two nucleotides. The "polynucleotide probe" provided herein is a nucleotide chain structure, as above, containing at least two nucleotides, at least one of which includes a modified nucleotide which has substantially the same structure as that given by Formula 1.

"Detectable label" refers to a moiety which accounts for the detectability of a complex or reagent. In general, the most common types of labels are fluorophores, chromophores, radioactive isotopes, and enzymes.

"Fluorophore" refers to a substance or portion thereof which is capable of exhibiting fluorescence in the detectable range. Typically, this fluorescence is in the visible region, and there are common techniques for its quantitation. Examples of fluorophores which are commonly used include fluorescein (usually supplied as fluorescein isothiocyanate [FITC] or fluorescein amine), rhodamine, dansyl and umbelliferone.

Formulae 2 through 5 illustrate the nucleotide numbering scheme used herein.

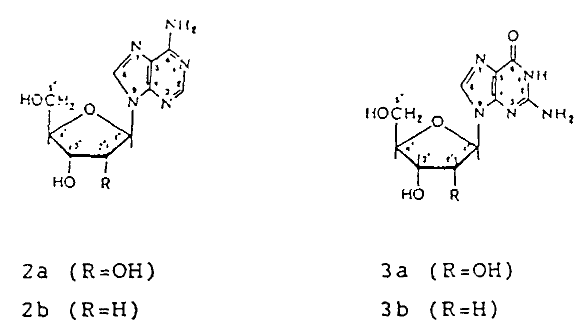

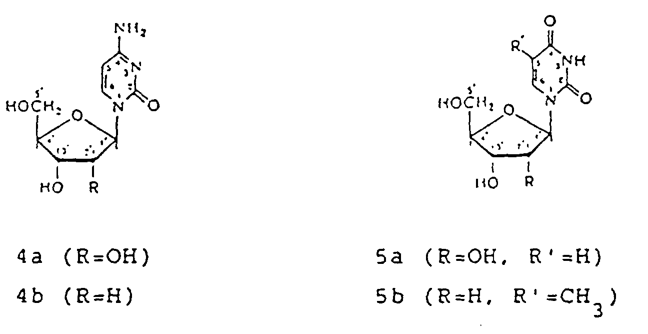

In a preferred embodiment, the substituents of the modified nucleotide of Formula 1 are as follows.

R¹, which is a reactive group derivatizable with a detectable label, is preferably -NH₂, -COOH or -SH.
R² is a linker moiety selected from 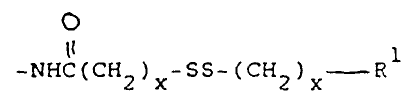 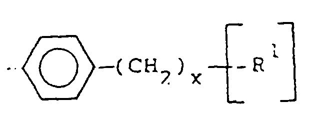 wherein x is an integer in the range of 1 and 8 inclusive.
R² is preferably a heterobifunctional linker such as those typically used to bind proteins to labels. In most cases, a free amino group on a protein or other structure will react with a carboxylic acid or activated ester moiety of the unbound R² compound so as to bind the linker via an amide linkage. Other methods of binding the linker to the nucleotide are also possible.

As may be seen in Formula 1, the linker, if present, is attached to the nucleotide structure through an alkylamine functionality -NH-(CH₂)ₓ- wherein x is an integer in the range of 1 and 8 inclusive, and the alkylamine functionality is present at the 4-position of the pyrimidine base.

As noted above, R³ is hydrogen, methyl, bromine, fluorine or iodine. Thus, the base of the nucleotide is a pyrimidine optionally substituted at the 5-position with the aforementioned R³ substituents.

R⁴ is typically hydrogen, if the modified nucleotide is a terminal 5' structure, or a suitable blocking group useful in polynucleotide synthesis. Examples of suitable blocking groups include substituted and unsubstituted aralkyl compounds, where the aryl is, e.g., phenyl, naphthyl, furanyl, biphenyl and the like, and where the substituents are from 0 to 3, usually 0 to 2, and include any non-interfering stable groups, neutral or polar, electron-donating or withdrawing, generally being of 1 to 10, usually 1 to 6 atoms and generally of from 0 to 7 carbon atoms, and may be an aliphatic, alicyclic, aromatic or heterocyclic group, generally aliphatically saturated, halohydrocarbon, e.g., trifluoromethyl, halo, thioether, oxyether, ester, amide, nitro, cyano, sulfone, amino, azo, etc.

In one or more steps during nucleotide chain synthesis, it may be desirable to replace the hydrogen atom or blocking group at the R⁴ position with a more stable, "capping" group. Suitable capping groups include acyl groups which provide for stable esters. The acyl groups may be organic or inorganic, including carboxyl, phosphoryl, pyrophosphoryl, and the like. Of particular interest are alkanoic acids, more particularly aryl-substituted alkanoic acids, where the acid is at least 4 carbon atoms and not more than about 12 carbon atoms, usually not more than about 10 carbon atoms, with the aryl, usually phenyl, substituted alkanoic acids usually of from 8 to 12 carbon atoms. Various heteroatoms may be present such as oxygen (oxy), halogen, nitrogen, e.g., cyano, etc. For the most part. the carboxylic acid esters will be base labile, while mild acid stable, particularly at moderate temperatures below about 50°C, more particularly, below about 35°C and at pHs greater than about 2, more particularly greater than about 4.

The modified nucleotide may also be attached to a support through the R⁴ position so as to facilitate addition of labeled or unlabeled nucleotides at the 3' (R⁵) position. In such a case, R⁴ is an anchoring group as will be described below. Covalent attachment to a support is also preferred during sample screening, as the time and complexity of separating the hybridized nucleotide chains from the sample is substantially reduced. When the modified nucleotide of Formula 1 is bound to one or more additional nucleotides at the 5' position, the R⁴ substituent is replaced with such additional nucleotides which are bound through their 3' phosphate groups.

R⁵, as noted, is hydrogen or a phosphorus derivative such as PO₃H₂, a phosphotriester, a phosphodiester, a phosphite, a phosphoramidite, an H-phosphonate or a phosphorothioate suitable for polynucleotide synthesis, which derivative enables sequential addition of nucleotides at the 3' position. More generally, such phosphorus derivatives are given by Formula 9 and Formula 10: 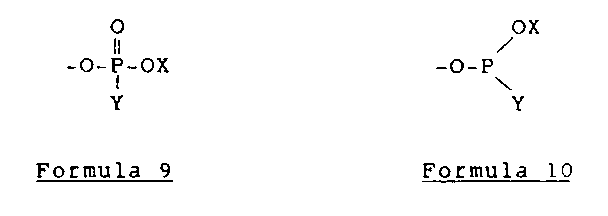 wherein X is preferably hydrogen or an aliphatic group. particularly a saturated aliphatic group, a β-heterosubstituted aliphatic group, where the β-substituent is an electron-withdrawing group which readily participates in β-elimination, either as the leaving group or the proton-activating group, substituted methylene, where the substituent may vary widely and supports a negative charge on the methylene through inductive or resonating effects; aryl; and aralkyl. Depending on the nature of the phosphorus functionality, one group may be chosen over another. Thus, depending upon whether a phorphorchloridite, phosphoramidite, phosphate, thiophosphate, phosphite, or the like, is employed, particular phosphoro ester groups will be preferred.

Similarly, the groups employed for Y will depend upon the nature of the phosphorus derivative employed for oligomerization. When the phosphoramidite is employed, Y will have the formula -NT¹T². where T¹ and T² may be the same or different and may be hydrocarbon or have from 0 to 5, usually 0 to 4 heteroatoms, primarily oxygen as oxy, sulfur as thio, or nitrogen as amino, particular tert.-amino, NO₂ or cyano. The two T's may be taken together to form a mono- or polyheterocyclic ring having a total of from 1 to 3, usually 1 to 2 heteroannular members and from 1 to 3 rings. Usually, the two T's will have a total of from 2 to 20, more usually 2 to 16 carbon atoms, where the T's may be aliphatic (including alicyclic), particularly saturated aliphatic, monovalent, or, when taken together, divalent radicals, defining substituted or unsubstituted heterocyclic rings. The amines include a wide variety of saturated secondary amines such as dimethylamine, diethylamine, diisopropylamine, dibutylamine, methylpropylamine, methylhexylamine, methylcyclopropylamine, ethylcyclohexylamine, methylbenzylamine, methylcyclohexylmethylamine, butylcyclohexylamine, morpholine, thiomorpholine, pyrrolidone, piperidine, 2,6-dimethylpiperidine, piperazine and similar saturated monocyclic nitrogen heterocycles.

R⁵ may also represent a point of attachment for one or more additional nucleotides at the 3' position. In that case R⁵ is phosphate, as such additional nucleotides are typically bound through a phosphate group.

As at the 5' position, the modified nucleotide may be attached to a support through the 3' position, i.e. through R⁵ . When the nucleotide thus attached to a support, R⁵ is an anchoring group as will be described below.

R⁶, in the case of deoxyribose, is H; in the case of ribose, is OH;, and, during RNA synthesis, is a suitable blocking group which protects the -OH moiety from modification. Blocking groups useful here generally include those given above for R⁴, and the specific choice of blocking group will be apparent to one skilled in the art. Examples of blocking groups which are preferred at the R⁶ position during RNA synthesis include silyl ethers such as t-butyldimethylsilyl, substituted methyl ethers, o-nitrobenzyl ether, esters such as levulinic ester, and the following pyranyl structures given by Formula 11 (tetrahydropyranyl) and Formula 12 (4-methoxytetrahydropyranyl):

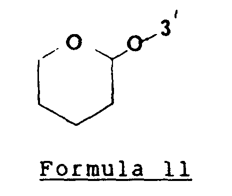

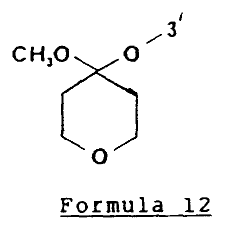

A particularly preferred blocking group is ortho-nitrobenzyl. Additional examples of suitable blocking groups may be found in Green, T.W., Protective Groups in Organic Synthesis, New York: Wiley & Sons, 1981.

The modified nucleotide will normally be derivatized with a label in a manner which will allow for detection of complex formation. A wide variety of labels may be used, and one or another label may be selected depending upon the desired sensitivity, the equipment available for measuring, the particular protocols employed, ease of synthesis, and the like. Labels which have found use include enzymes, fluorescers, chemiluminescers, radionuclides, enzyme substrates, cofactors or suicide inhibitors, specific binding pair members, particularly haptens, or the like. The molecule involved with detection may be covalently bound to the modified nucleotide or indirectly bound through the intermediacy of a specific binding pair, i.e. ligand and receptor. Examples of ligands and receptors include biotin-avidin, hapten-antibody, ligand-surface membrane receptor, metal-chelate, etc.

As suggested above, it is preferred that the modified nucleotide be covalently bound to a support at either the R⁴ or R⁵ positions for oligonucleotide synthesis. A wide variety of supports may be used, including silica, Porasil C, polystyrene, controlled pore glass (CPG), kieselguhr, poly(dimethylacrylamide), poly(acrylmorpholide), polystyrene grafted onto poly(tetrafluoroethylene), cellulose, Sephadex LH-20, Fractosil 500, etc.

Depending on the nature of the support, different functionalities will serve as anchors. As noted above, these "anchoring" groups are at either the 3' or the 5' position, i.e. at either the R⁵ or R⁴ positions, respectively. For silicon-containing supports, such as silica and glass, substituted alkyl or aryl silyl compounds will be employed to form a siloxane or siloximine linkage. With organic polymers, ethers, esters, amines, amides, sulfides, sulfones and phosphates may find use. For aryl groups, such as polystyrene, halomethylation can be used for functionalization, where the halo group may then be substituted by oxy, thio (which may be oxidized to sulfone), amino, phospho (as phosphine, phosphite or phosphate), silyl or the like. With a diatomaceous earth element (e.g., kieselguhr), activation may be effected by a polyacrylic acid derivative and the active functionality reacted with amino groups to form amine bonds. Polysaccharides may be functionalized with inorganic esters, e.g. phosphate, where the other oxygen serves to link the chain. With polyacrylic acid derivatives, the carboxyl or side chain functionality, e.g., N-hydroxyethyl acrylamide, may be used in conventional ways for joining the linking group.

The modified nucleotide of Formula 1, as previously suggested, can be used as a substrate for synthesis of polynucleotide probes. Additional nucleotides may be sequentially added at the 5' position by, for example, the phosphoramidite method of Beaucage and Caruthers, Tetrahedron Lett. 22(20):1859-62 (1981) or the phosphotriester method of Itakura, J. Biol. Chem. 250:4592 (1975), or the like, or at the 3' position by Belagaje and Brush, Nuc. Acids Research 10:6295 (1982), or both. The nucleotides which are sequentially added may be unlabeled, or they may be modified according to Formula 1 and derivatized with a label at the R¹ moiety. Accordingly, one or more labels may be present within a polynucleotide chain rather than at one end.

This polynucleotide probe includes at least one modified nucleotide having substantially the same structure as that given by Formula 1, i.e. including at least one modified nucleotide having the structure given by Formula 13: 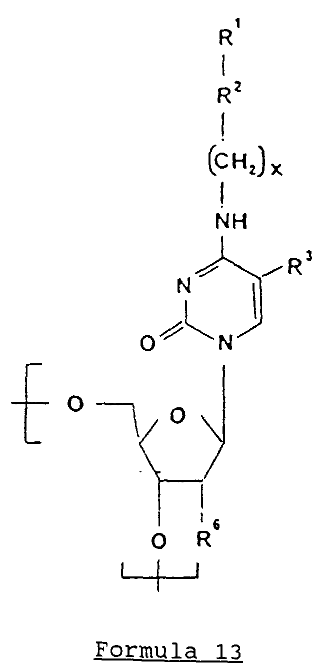 wherein R¹ is a reactive group derivatized with a detectable label. R² is a linking moiety selected from 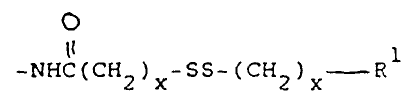 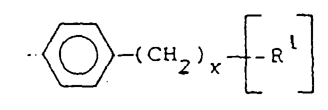 wherein x is an integer in the range of 1 and 8 inclusive.
R³ is selected from the group consisting of hydrogen, methyl, bromine, fluorine and iodine, R⁶ is H, OH, or OR where R is an acid-sensitive, base-stable protecting group and x is an integer in the range of 1 and 8 inclusive. The polynucleotide probe may have a single label or a plurality of labels, depending upon the nature of the label and the mechanism for detection. Where the label is fluorescent, for example, a distance of at least 3 to 12 Angstroms should be maintained between fluorescent species to avoid any fluorescence quenching.

Such labeled polynucleotide probes may be used in the assays described in applicants' co-pending application Serial No. 807,624, or in any number of other applications, including conjugation with enzymes, antibodies and solid supports. An example of one such use of applicants' novel oligonucleotide probes is in the detection of a known sequence of DNA. The probe may be prepared so as to be attached, for example, to a standard latex solid support or to an avidin support in the case of biotin-labeled probes. Sample containing single-stranded or double-stranded DNA sequences to be analyzed is caused to contact the probe for a time sufficient for hybridized nucleic acid complexes to form, and any such complexes are detected by means of the fluorescent, biotin or otherwise detectable label.

Synthesis of the modified nucleotide: The present invention also relates to a method of synthesizing the novel modified nucleotide of Formula 1. In the preferred embodiment, a pyrimidine nucleotide is provided which has the structure of Formula 14: 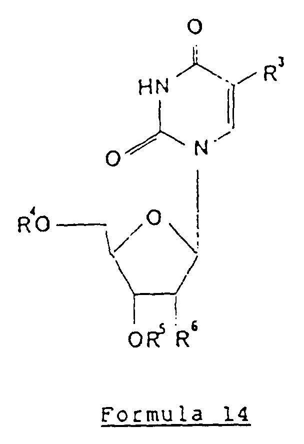 wherein R³ is as given above, R⁴ and R⁵ are hydrogen, and R⁶ is OH or H. The 5' position of the sugar ring -- and the 2' position as well if the sugar is ribose rather than deoxyribose -- is then protected against modification during subsequent reaction steps by addition of a dimethoxytrityl group (see Example 3) or other suitable protecting group, the addition reaction allowed to proceed for a time sufficient to ensure substantial completeness. Similarly, the 3' hydroxyl group is protected with a silyl or other suitable functionality (see Example 4).

Examples of particularly suitable protecting groups include those set forth above as "R⁶". i.e., substituted methyl ethers, esters, pyranyls and the like.

When the nucleoside is thymine or uracil, or uracil modified at the 5-position by an R³ substituent, i.e. a pyrimidine or substituted pyrimidine which has an oxy rather than an amino substituent at the 4-position, the carbonyl is converted to an amine moiety by, for example, reaction with an activating agent such as 1-(mesitylene-2-sulfonyl)-tetrazole (MS-tet) or other suitable condensing reagent. Activating agents for use herein also include other sulfonyl compounds given by the formula E₁-SO₂-E₂ wherein E₁ is tetrazoyl. nitrotriazoyl, triazoyl, imidazoyl, nitroimidazoyl, or the like, and E₂ is an aryl or substituted aryl group such as mesitylene, etc. Another class of suitable activating agents is given by Formula 16: 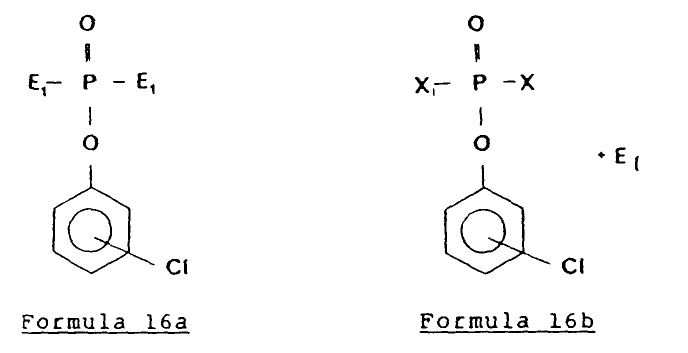 wherein E₁ is as defined above, and X is a halogen substituent, preferably chlorine. In Formula 16b, E₁ is present in a solution containing the activating agent but is not bound thereto. In general, any activating agent may be used and may include one or more halogen substituents, preferably chlorine, on the ring structure which after reaction can be displaced by ethylene diamine or like reagent. This conversion is followed by reaction with an alkyldiamine such as ethylenediamine to give a nucleotide having a -NH-(CH₂)ₓNH₂ functionality at the 4-position of the pyrimidine ring (see Examples 5, 6). The free amine group so provided is then optionally reacted with caproic acid, an activated caproic acid ester, or with a caproic acid derivative such as 6-aminocaproic acid, in order to ensure sufficient spacing between the nucleotide and the detectable label to be attached at the R¹ moiety. The caproic acid or related compound may be labeled prior to attachment (see Example 7) or subsequently.

The modified nucleotides of the present invention may also be synthesized by methods in which a pyrimidine nucleotide is provided which has the structure of Formula 15: 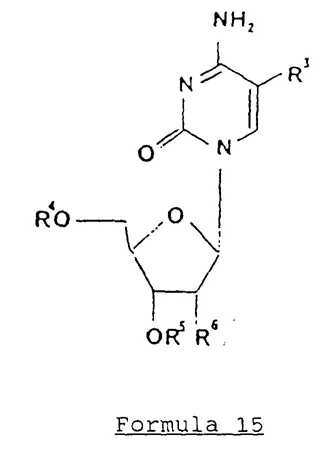 wherein R³ is as given above, R⁴ and R⁵ are hydrogen, and R⁶ is OH or H. The 5' position of the sugar ring -- and the 2' position as well if the sugar is ribose rather than deoxyribose -- is then protected against modification during subsequent reaction steps by addition of a dimethoxytrityl group (see example 3) or other suitable protecting group, the addition reaction allowed to proceed for a time sufficient to ensure substantial completeness. Similarly, the 3' hydroxyl group is protected with a silyl or other suitable functionality (see Example 4).

Examples of particularly suitable protecting groups include those set forth above as "R⁶", i.e., substituted methyl ethers, esters, pyranyls and the like.

When the nucleotide is cytosine or a 5-modified cytosine, i.e. substituted with an R³ other than hydrogen, the exocyclic amino functionality can be converted to an N⁴-aminoalkyl or N⁴-aminoaryl cytosine by reaction with an aryl sulfonyl chloride followed by reaction with an alkyl- or aryldiamine (Scheme I). See, e.g., Markiewicz. W.T. and R. Kierzek, 7th Intl. Round table, pp. 32 and 72 (1986). Alternately, preparation of N⁴-substituted cytosine may be effected using a bisulfite-catalyzed exchange reaction (Scheme II). See Schulman, L.H. et al., Nuc. Acids Res. 9:1203-1217 (1981) and Draper, D.E., Nuc. Acids Res. 12:989-1002 (1984).

Alternatively, where the alkylamine group is more than about 6 carbon atoms long, the free amine group thereof may directly bond to a suitable detectable label.

The synthesis may further include removal of the dimethoxytrityl or other protecting groups with acid, followed by, if desired, phosphorylation or phosphitylation of the 3' position in preparation for sequential addition of nucleotides.

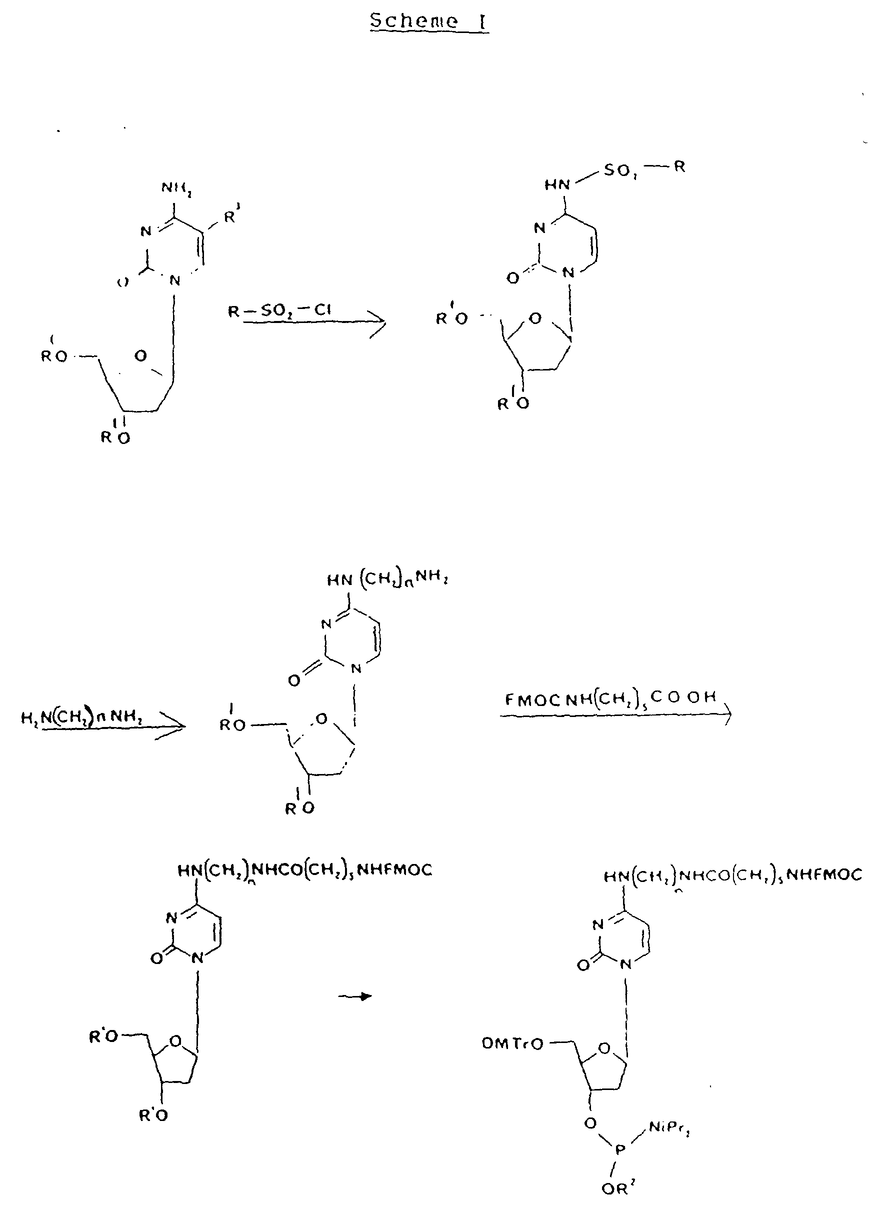

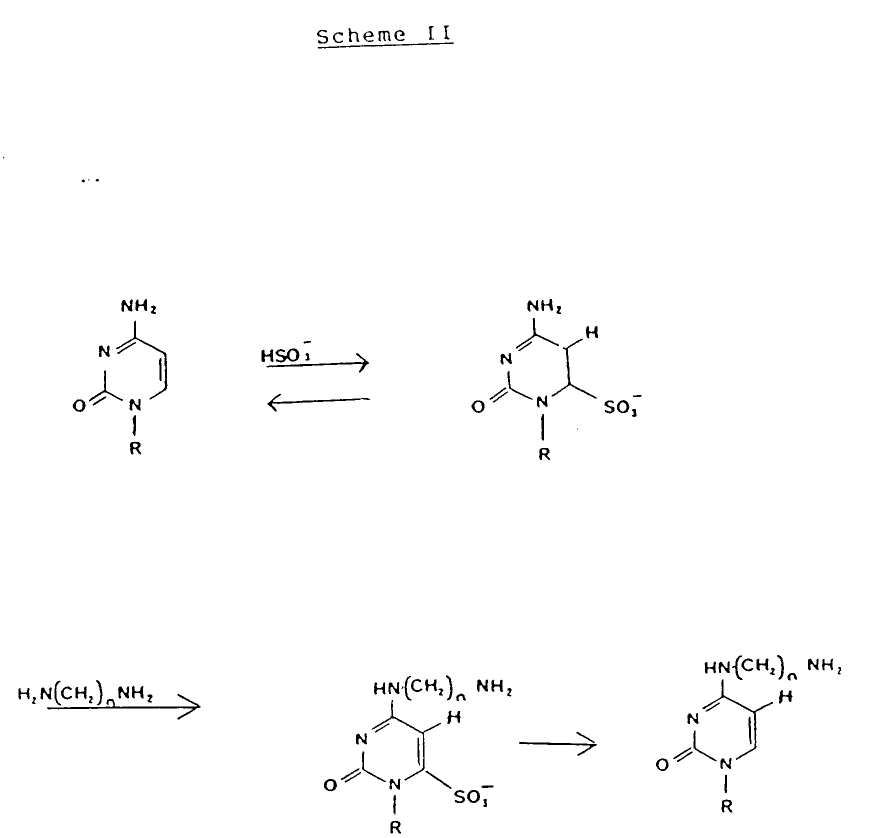

It is to be understood that the foregoing description as well as the Examples which follow are intended to illustrate and not limit the scope of the invention. Other aspects, advantages and modifications within the scope of the invention will be apparent to those skilled in the art to which the invention pertains.

The following Examples do not illustrate modified nucleotides falling within the claims and are included for comparative purposes only. However, the Examples do describe synthetic procedures applicable to the modified nucleotides of the invention.

### Experimental

### Analyte BglII HBV Fragment

GATCTCC | TAGACACCGCCTCAGCTCTGTATCGAGAAG | CCT | TAGAGTCTCCTGAG
AGG | ATCTGTGGCGGAGTCGAGACATAGCTCTTC | GGA | ATCTCAGAGGACTC
→ 1 ← → 2

CATTGCTCACCTCACC | ATA | CTGCACTCAGGCAAGCCATTCTCTGCTGGG | GGG | AATTGATG
GTAACGAGTGGAGTGG | TAT | GACGTGAGTCCGTTCGGTAAGAGACGACCC | CCC | TTAACTAC
← → 3 ← →

ACTCTAGCTACCTGGGTGGGTA | ATA | ATTTGGAAGATCCAGCATCTAGGGATCTTG | TAG | TA
TGAGATCGATGGACCCACCCAT | TAT | TAAACCTTCTAGGTCGTAGATCCCTAGAAC | ATC | AT
4 ← → 5 ← →

AATTATGTTAATACTAACGTGGGTTTAA | AGA | TCAGGCAACTATTGTGGTTTCATATATCT
TTAATACAATTATGATTGCACCCAAATT | TCT | AGTCCGTTGATAACACCAAAGTATATAGA
6 ← → 7

T | GCC | TTACTTTTGGAAGAGAGACTGTACTTGAAT | ATT | TGGTCTCTTTCGGAGTGTGGATT
A | CGG | AATGAAAACCTTCTCTCTGACATGAACTTA | TAA | ACCAGAGAAAGCCTCACACCTAA
← → 8 ← → 9

CGCACTC | CTC | CAGCCTATAGACCACCAAATGCCCCTATCT | TAT | CAACACTTCCGGAAACT
GCGTGAG | GAG | GTCGGATATCTGGTGGTTTACGGGGATAGA | ATA | GTTGTGAAGGCCTTTGA
← → 10 ← → 11

ACTGTTGTTAGAC | GAC | GGGACCGAGGCAGGTCCCCTAGAAGAAGAA | CTCCCTCGCCTCGC
TGACAACAATCTG | CTG | CCCTGGCTCCGTCCAGGGGATCTTCTTCTT | GAGGGAGCGGAGCG
← → 12 ←

AGACGCA
TCTGCGTCTAG

→ ← indicates probed segments

### Labelling and Capturing Probe Sets (Refer to Fig. 1)

3' GACTTGCAAGTTGGTCAAGT|ATCTGTGGCGGAGTCGAGACATAGCTCTTC| 5'
A → 1 ←

GTGGTGAAAGAGGTTTCTTC|ATCTCAGAGGACTCGTAACGAGTGGAGTGG|
B → 2 ←

GACTTGCAAGTTGGTCAAGT|GACGTGAGTCCGTTCGGTAAGAGACGACCC|
A → 3 ←

GTGGTGAAAGAGGTTTCTTC|TTAACTACTGAGATCGATGGACCCACCCAT|
B → 4 ←

GACTTGCAAGTTGGTCAAGT|TAAACCTTCTAGGTCGTAGATCCCTAGAAC|
A → 5 ←

GTGGTGAAAGAGGTTTCTTC|ATTTAATACAATTATGATTGCACCCAAATT|
B → 6 ←

GACTTGCAAGTTGGTCAAGT|AGTCCGTTGATAACACCAAAGTATATAGAA|
A → 7 ←

GTGGTGAAAGAGGTTTCTTC|AATGAAAACCTTCTCTCTGACATGAACTTA|
B → 8 ←

GACTTGCAAGTTGGTCAAGT|ACCAGAGAAAGCCTCACACCTAAGCGTGAG|
A → 9 ←

GTGGTGAAAGAGGTTTCTTC|GTCGGATATCTGGTGGTTTACGGGGATAGA|
B → 10 ←

GACTTGCAAGTTGGTCAAGT|GTTGTGAAGGCCTTTGATGACAACAATCTG|
A → 11 ←

GTGGTGAAAGAGGTTTCTTC|CCCTGGCTCCGTCCAGGGGATCTTCTTCTT|
B → 12 ←

| → Labeled Probe ← | → Probe Segments to HBV ← |
recognition sequences

A - Fluorescein label conjugate binding site
B - Biotin or DNA conjugate binding site

Label conjugate (A') for DNA or avidin support:

Fluorescein - 5' CTGAACGTTCAACCAGTTCA 3'

DNA sequence (B'C) bound to solid support:

3' GAAGAAACCTCTTTCACCACTGTCATCAAAAGGTTAACCATGTTTCTTGT 5'

Biotin conjugate (B') for avidin support:

Biotin - 5' CACCACTTTCTCCAAAGAAG 3'

Preparation of biotin or fluorescein labeled DNA (A' or B'):

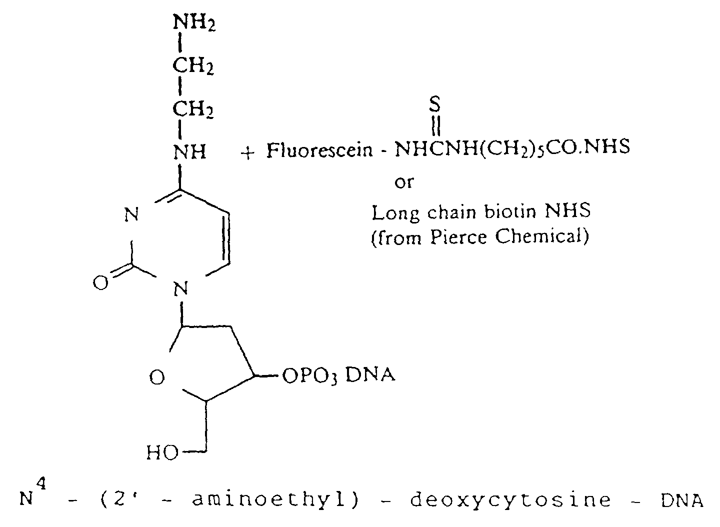

The analyte is an HBV BgIII fragment as indicated above. (Valenzuela et al. (1981) in Animal Virus Genetics, eds. Fields, B., Jaenisch, R., Fox, C.F., Academic Press, Inc., N.Y., pp 57-70.) A subset of labeling and capturing probes are indicated, where 12 different sequences complementary to different sequences present in HBV are provided. Six of the HBV complementary sequences are joined to a common sequence (A) for complexing with the label conjugate (A'). The other six HBV complementary sequences are joined to a common sequence (B) for complexing with a biotinylated sequence (B') or a third DNA sequence (B'C) for binding to a support. In Figure 1 is shown an illustration of the final complex involving the HBV strand and the various reagents.

### Example 1

### Labeling of Caproic Acid Derivative

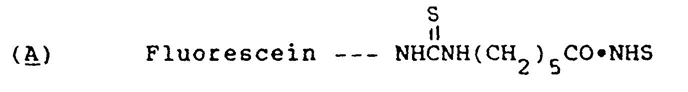

To 1 mmole of fluorescein isothiocyanate in 5 ml of DMF was added 2 mmole of 6-aminocaproic acid and 540 µl of triethylamine. After 24 h at room temperature, the product was isolated by preparative thin layer chromatography (Warner and Legg, Inorg. Chem. 18:1839 (1979)). The dried product was suspended in 10 ml of 1:1 DMF/THF (v/v) to which 1.5 mmole of N-hydroxy succinimide and 1 mmole of dicyclohexylcarbodiimide were added. After 18 h at room temperature the solution was filtered through glass wool and diluted to a 0.2M final concentration of A with DMF (assuming a 100% yield from step 1).

### Example 2

### 6-N⁴-(2-Aminoethyl)- Deoxycytidine

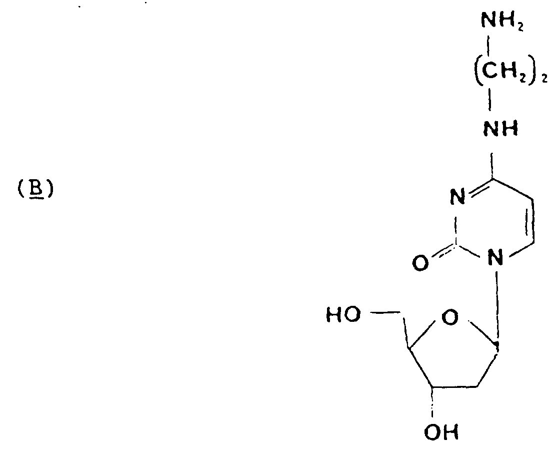

An alkylated derivative of deoxycytidine, N⁴-(2-aminoethyl) deoxycytidine (B) was prepared from properly protected deoxyuridine via the 4-tetrazoyl derivative as described by Reese and Ubasawa, Tetrahedron Lett. 21:2265 (1984). This latter derivative was converted to B by displacement of the tetrazoyl moiety with ethylene diamine essentially as described by Sung, J. Org. Chem. 47:3623 (1982) and Maggio et al., Tetrahedron Lett. 25:3195 (1984). The corresponding 5'-DMT-3'-phosphoramidite N⁴-(2-N-trifluoroacetylaminoethyl) deoxycytidine was prepared by blocking the alkylamine with trifluoroacetic anhydride and then preparing the corresponding N,N-diisopropyl phosphoramidite as described (Beaucage and Caruthers, supra; McBride and Caruthers, Tetrahedron Lett. 24:245 (1983)).

### Example 3

### Probe Preparation (Fluorescein Label)

Synthetic oligonucleotides were prepared by an automated phosphoramidite method as described in Warner et al., DNA 3:401 (1984). Purification was carried out according to Sanchez-Pescador and Urdea, DNA 3:339 (1984).

The aminoethyl derivative of deoxycytidine as prepared in Example 2 was incorporated by standard coupling procedures during the oligonucleotide synthesis and the purified modified oligonucleotides were used for incorporation of a fluorescein label as follows. To a dried sample (3-5 OD 260 units) of the aminoethyl deoxycytidine containing oligomer were added 50 µl of DMF and 25 µl of the 0.M2 stock solution of A described above. After 18 h at room temperature, the solution was partially purified by Sephadex G-10 chromatography eluted with water, dried and further purified by polyacrylamide gel, as above.

### Example 4

### Probe Preparation (Biotin Label)

Using the probes containing aminoethylcytidine as prepared in the previous example, biotin labeling was achieved as follows. The oligonucleotide (3-5 OD 260 units) was taken up in 50 µl 0.M1 sodium phosphate, pH 7.0 and 50 µl of DMF to which 100 µl of a DMF solution containing 1 mg of a "long chain" N-hydroxysuccinimidyl biotin (Pierce Chemical) was added. After 18 h at room temperature, the biotinylated probe was purified as described for the fluorescein labeled probe.

### Example 5

### Preparation of Solid-Supported DNA Probe

Fragment B'C (a synthetic 50mer) was 5'-phosphorylated with T4-polynucleotide kinase and ATP using standard conditions. After gel purification as described above, the oligonucleotide was dried by evacuation.

Hydroxylated latex (10mg; 0.8µ; Pandex Laboratories) was washed with DMSO, then three portions of 40mM MES (morpholinoethanesulfonic acid), pH 6.0 by centrifugation. 1500pmoles of 5'-phosphorylated fragment B'C was taken up in 90ml of 40mM MES and added to the washed support. A solution was prepared to contain 100mg of EDAC in 100ml of MES. After adding 5µl of the EDAC solution and mixing, the reaction mixture was evaporated until 30µl total remained. The mixture was left at 37°C for 18h, then centrifuged for 2min at 12,000rpm. The supernatant was discarded. The latex was suspended in 30ml of DMSO, vortexed, 100µl of water was added, the mixture vortexed for 2min and the supernatant was discarded after centrifugation. This washing process was repeated twice. The support was then washed three times with 100ml portions of 4xSSC, H₂O, then H₂O at 37°C for 15min (yield 20 picomoles fragment B'C per mg of latex).

### Example 6

### Assay for HBV DNA Usinq DNA Solid Support

A pBR322 clone containing the entire HBV genome (Valenzuela et al., Animal Virus Genetics, R. Jaenisch, B. Fields and C.F. Fox, Eds. (Academic Press: New York) pp. 57-70 (1980)) was cut with BglII and used as the analyte nucleic acid. Analyte in 10ml of formamide containing 6 picomoles of the labeling and capturing probe sets was heated to 95°C for 10min and cooled to room temperature. To this mixture, 60µl of water, 20µl of 20xSSC, 10ml of 1% NP40 and 2µl (10µg) of polyA are added, vortexed and incubated at 37°C for lh.

The solid supported DNA probes (8 picomoles 400vg) is added and incubated for an additional 1.5h. The mixture is centrifuged at 12,000rpm for 2min and the supernatant discarded. The support is washed once by vortexing the pellet into solution with 100ml of 4xSSC, followed by centrifugation. To the washed beads are added a mixture of 4ml of 20xSSC, 2µl of 1% NP4O, 1µl (5µg) polyA, 13µl of water and 6 picomoles of fluorescein labeled probe. After incubation at 37°C for 30min, the beads are transferred to a Pandex filter plate, washed four times with 100ml of 4xSSC by vacuum filtration on the 0.2µ cellulose acetate membrane of the plate. The sample is vacuumed to dryness and read on the fluorescein channel A (λ_{excitation} -485; λₑₘᵢₛₛᵢₒₙ -525) of the Pandex screen machine.

**TABLE 1**

| Condition | Fluorescence Counts (Average of 4) |
|---|---|
| 0.5 pmole HBV | 5062 +/- 345 |
| 0.25 pmole HBV | 4117 +/- 262 |
| No Analyte | 3197 +/- 520 |
| No Biotinylated Probe | 3856 +/- 642 |

### Example 7

### Assay for HBV DNA Usinq Avidin Support

### Experiment 7a:

Analyte was mixed and incubated with the labeling and capturing probes as above. Biotin labeled probe (12 picomoles) in 5µl H₂O was then added, vortexed and incubated at 37°C for 30min. To the mixture, 20ml of a 0.25% (w/v) 0.8µ avidin latex (Pandex Laboratories) in 1xPBS is added and incubated at 37°C for 1h. The mixture is washed, incubated with fluorescein probe, washed and read on the Pandex screen machine as described above.

**TABLE 2**

| Condition | Fluorescence Counts (Average of 4) |
|---|---|
| 0.5 picomole HBV | 4052 +/- 462 |
| 0.25 picomole HBV | 2644 +/- 397 |
| 0.10 picomole HBV | 1956 +/- 173 |
| No Analyte | 1641 +/- 370 |
| No Biotinylated Probe | 1631 +/- 474 |

### Experiment 7b:

The HBV plasmid was sonicated to an average size of 500bp. The denaturation and hybridization were carried out as above except that 30 picomoles of labeling and capturing probes were used and a 5h annealing was employed. After incubation with 30 picomoles of biotinylated probe (2h), 50µl of 0.25% avidin beads were added and incubated (1.5h). A fluorescein probe was added and incubation was carried out for 1h followed by washing and reading on the Screen Machine as described above.

**TABLE 3**

| Condition | Fluorescence Counts (Average of 4) |
|---|---|
| 0.5 picomole HBV | 5748 +/- 244 |
| 0.4 picomole HBV | 5352 +/- 331 |
| 0.3 picomole HBV | 4716 +/- 243 |
| 0.2 picomole HBV | 4071 +/- 243 |
| 0.1 picomole HBV | 3320 +/- 27! |
| No Analyte | 1679 +/- 167 |
| No Biotinylated Probe | 1716 +/- 177 |

It is evident from the above results that a highly specific sensitive assay for specific nucleic acid sequences is provided. Reagents can be readily prepared to the sequence of interest and with a few simple manipulative steps, the presence or absence of a sequence in a sample determined. The method is versatile in permitting a wide variety of labels which can be readily determined by conventional equipment. Probes can be synthesized to the desired length and easily linked to the label or a support. Universal sequences can be prepared for the label and binding to the support. Various protocols may be employed where more or less rigorous removal of background interference is achieved depending upon the requirements of the assay.

### Example 8

### 5'-Dimethoxytrityl-2'-Deoxyuridine

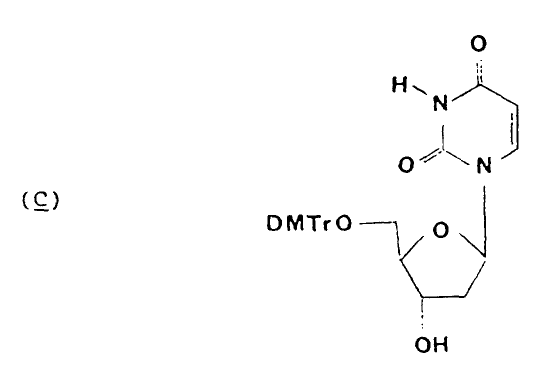

To 2-Deoxyuridine (10 g, 44 mmole) dried by coevaporation of pyridine and suspended in pyridine (100 ml) was added 18.4 g (54 mmole) 4,4'-dimethoxytrityl chloride (DMT-Cl). The reaction was allowed to proceed for 18 h at room temperature, and 100 ml methanol was added to deactivate excess DMT-Cl. Most of the pyridine was then removed in vacuo, and the residue, dissolved in 500 ml ethyl acetate, was washed with saturated aqueous NaHCO₃ (3x500 ml). The organic phase was dried over solid Na₂SO₄ and evaporated to dryness. The residue was purified by flash chromatography on silica gel to give 18.0 g (77%) of 5'-dimethoxytrityl-2'-deoxyuridine (C).

### Example 9

### 5'-O-(4,4'-Dimethoxytrityl)-3'-t-Butyldimethylsilyl-2'-Deoxyuridine

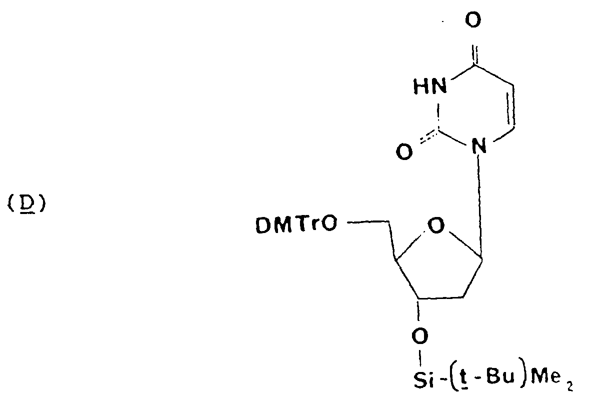

To 18 g (34 mmole) of C in 200 ml DMF was added imidazole (5.8 g, 85 mmole) with rapid stirring to assure complete dissolution. t-Butyldimethylsilyl chloride (7.65 g, 51 mmole) dissolved in a small volume of DMF was added dropwise with stirring and the reaction was allowed to proceed in the dark for 18 h at room temperature. The reaction mixture was diluted with ethyl acetate (250 ml) and extracted with NaHCO₃ (3x250 ml). The organic phase was dried over Na₂SO₄ and evaporated to dryness. The residue was purified by flash chromatography on silica gel to give 15.0 g (68% yield) of 5'-O-(4,4'-dimethoxytrityl-3'-t-butyldimethylsilyl-2'-deoxyuridine (D).

### Example 10

### 4-(1,2,3,4-Tetrazol-1-yl)-[5'-(4,4'-Dimethoxytrityl)-3'-t-Butyldimethylsilyl-β-D-2'-Deoxyribosyl] Pyridine-2(1H)-one

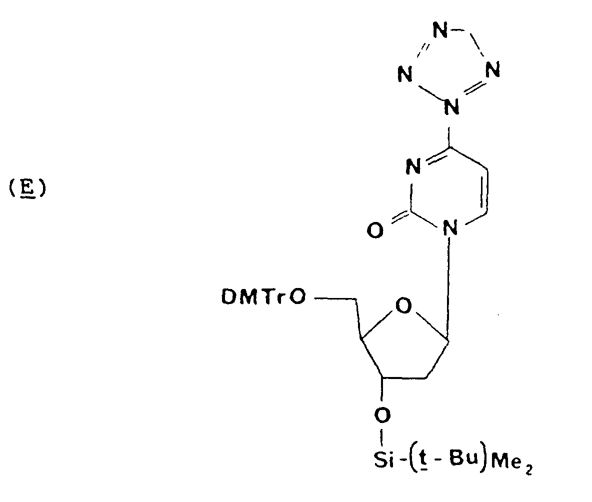

To 15.0 g (23 mmole) of D, dried by coevaporation of pyridine and dissolved in pyridine (50 ml) was added diphenylphosphate (2.9 g, 11.5 mmole) dissolved in pyridine (5 ml). 1-(Mesitylene-2-sulfonyl)-tetrazole (MS-tet) (15.5g, 61.5 mmole) dissolved in pyridine (45 ml) was added and the reaction mixture allowed to proceed in the dark for 18 h at room temperature. To the dark brown reaction mixture was added 25 ml water. After 30 min, the product was concentrated under reduced pressure. The residue was dissolved in 250 ml methylene chloride, washed with an aqueous NaHCO₃ solution (3x250 ml), dried over Na₂SO₄, and the solvent was removed under reduced pressure in the presence of toluene. The residue was purified by flash chromatography on silica gel to give 10.0 g (62%) of 4-(1,2,3,4-Tetrazol-1-yl)-[5'-(4,4'-dimethoxytrityl)-3'-t-butyldimethylsilyl-β-D-2'-deoxy-ribosyl]-pyridine-2(1H)-one (E).

### Example 11

### 4-N-(2-Aminoethyl)-5'-Dimethoxytrityl-3'-t-Butyldimethylsilyl-2'-Deoxycytidine

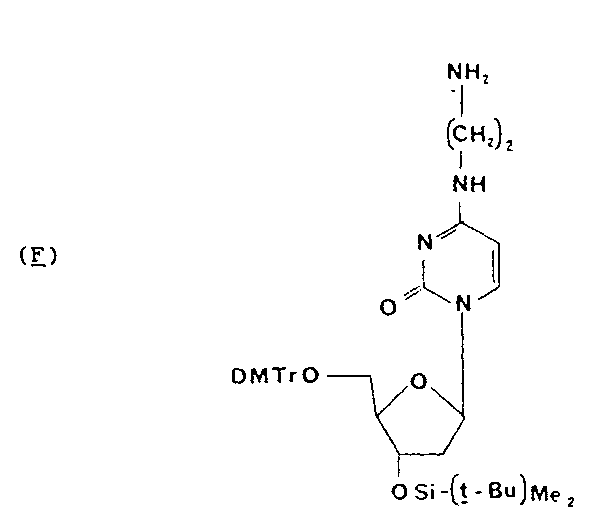

To a solution of ethylene diamine (9.3 ml, 143 mmole) in dioxane (100 ml) cooled to 5°C was added E (10.0 g, 14.3 mmole) and left for one hour. The solvent was removed at reduced pressure and the residue was coevaporated with toluene to remove excess ethylene diamine. The product was purified by chromatography on a silica gel column, eluted with 12-20% methanol in methylene chloride to give 7.15 g (75%) of 4-N-(2-aminoethyl)-5'-dimethoxytrityl-3'-t-butyldimethylsilyl-2'-deoxycytidine (F). The product was shown to react positively with ninhydrin, confirming the presence of a free amine moiety.

### Example 12

### N⁴-(N-FMOC-6-Aminocaproyl-2-Aminoethyl)-5'-Dimethyltrityl-3'-t-Butyldimethylsilyl-2'-Deoxycytidine

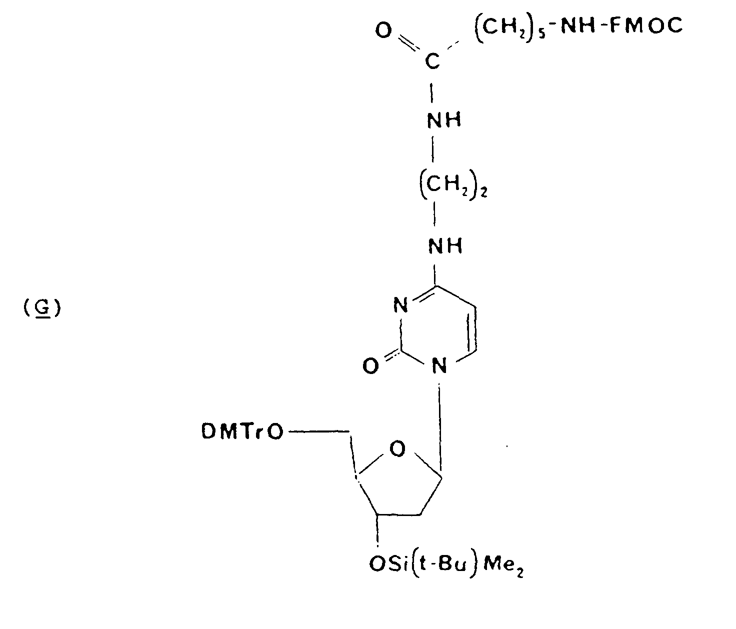

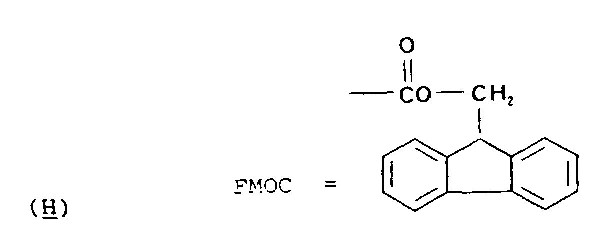

To a solution of F (6.5 g, 9.6 mmole) in pyridine (50 ml) was added N-FMOC-6-aminocaproic acid (4.26 g, 12 mmole) (FMOC represented by structure H) and DCC (2.96 g, 14.4 mmole). After 3 h, the reaction was complete as judged by tlc (silica in 10% methanol/ methylene chloride). Pyridine was removed at reduced pressure. The residue was extracted with ethyl acetate, insoluble dicyclohexylurea (DCHU) filtered off and the solvent removed. The product was isolated by silica gel chromatography eluted with 4% methanol in methylene chloride affording 7.3 g (70%) of N⁴-(N-FMOC-6-amino-caproyl-2-amino-ethyl)-5'-dimethyltrityl-3'-t-butyldimethylsilyl-2'-deoxycytidine (G).

### Example 13

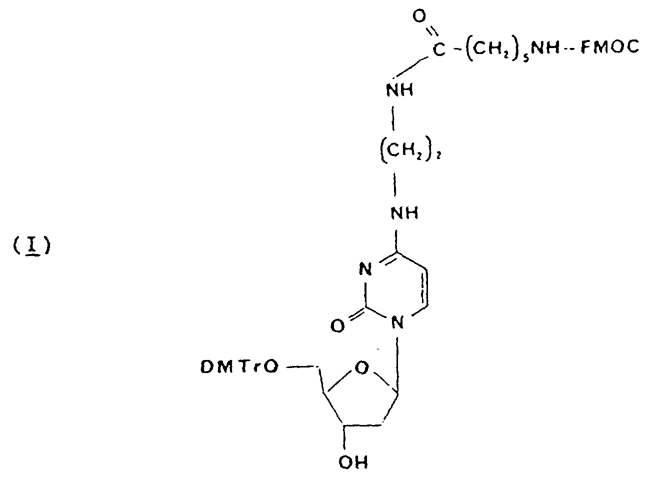

A solution of tetrabutylammonium fluoride (15 mmole, 15 ml of a 1M solution in THF) and aqueous HF (1.05 ml of a 50% aqueous solution) were mixed and dried by coevaporation of pyridine. The residue was dissolved in pyridine (15 ml) and added to G (7.2 g, 7.3 mmole) which was dissolved by sonication. After 18 hours at 4°C the reaction mixture was diluted with 200 ml methylene chloride. Concentrated aqueous NaHCO₃ was carefully added followed by solid NaHCO₃, added gradually so as to neutralize the HF/pyridine. After drying over Na₂SO₄, the organic phase was concentrated to an oil, which was subjected to silica gel chromatography. The product N⁴-(N-FMOC-6-amino caproyl-2-aminoethyl)-5'-dimethoxytrityl-'-deoxycytidine (I) was eluted with 5-6% methanol in methylene chloride to give an 86% yield (6.0 g).

### Example 14

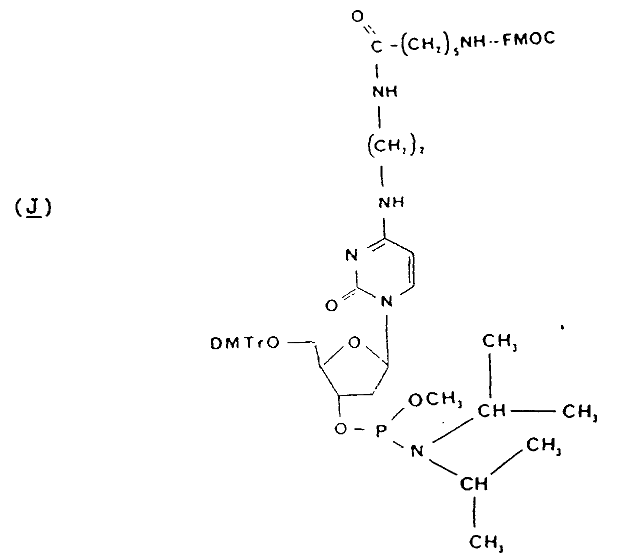

To 5.1 g (5.7 mmole) of I in methylene chloride containing (diisopropylethylamine) was added 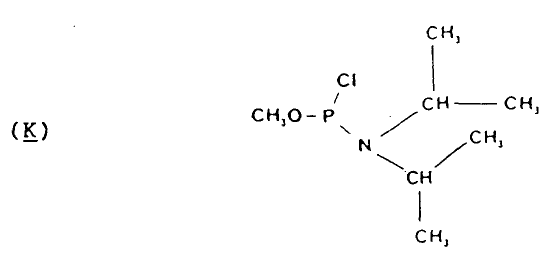 (chloro-N,N-diisopropylaminomethoxy phosphine, 1.3 ml [1.2 eq.], K) at 0°C under argon. After 1 hr, ethyl acetate (200 ml) was added and washed with 80% saturated aqueous sodium chloride; after drying of the organic phase over Na₂SO₄, the product in methylene chloride was added dropwise to hexane at -40°C to precipitate 4.43 g (75%) of J.

### Example 15

### Synthesis of Horseradish Peroxidase (HRP): DNA Conjugates

Sequence 1 (5'-[LCA]CTGAACGTTCAACCAGTTCA-3') where LCA = N⁴(6-aminocaproyl-2-aminoethyl)-deoxy cytidine) was synthesized chemically and purified as described elsewhere (Warner, et al. (1984) DNA 3, 401). To 10 OD 260 units dissolved in 50 µl of water were added 10 µl of 1.0 M sodium borate, pH 9.3, and 500 µl of distilled dimethylformamide containing 20 mg of p-phenylene diisothiocyanate. The solution was vortexed and set for 2 hr at room temperature in the dark. Approximately 3 ml of n-butanol was then added. After vortexing, adding 3 ml of water, and vortexing again, the tube was centrifuged and the yellowish upper layer discarded. The extraction process was repeated with subsequent n-butanol additions until an final volume of approximately 50 µl was obtained. The butanol was removed by evacuation, then 10 mg of HRP in 200 µl of 0.1 M borate, pH 9.3, was added. The mixture was vortexed, then set at room temperature overnight in the dark.

Separation of the HRP-DNA conjugate from free enzyme and DNA was achieved on a 7% polyacrylamide gel. The 250 µl reaction mixture was quenched with 100 µl of 25% glycerol, 0.5% SDS. 0.5% bromophenol blue, 2.5 mM EDTA. The solution was then distributed into 10 lanes of a 20 x 20 0.15 cm gel and run at 60 mAmps under standard conditions (Maxam, A., and Gilbert, W., (1980) Methods in Enzymol 65, 499-560) until the bromophenol blue was about 2/3 down the gel. The gels were set on Baker F-254 silica 60 plates that had been covered with Saran Wrap (Dow) and examined with a handheld UV-short wavelength lamp held above. Pictures of the UV-shadowed bands were taken with a Polaroid MP-4 camera system fitted with a Kodak No. 59 green filter, after which the bands were cut out with a razor blade. The bands were put into a 10-ml Bio-Rad polypropylene econo-columns to which 3 ml of 0.1 M sodium phosphate, pH 7.5, was added, then set at room temperature overnight.

The contents of the column were filtered through the frit at the column bottom into an Amicon Centricon microconcentrator that had been washed twice with distilled water. The HRP-DNA conjugate was then concentrated by centrifugation at 3500 rpm and washed twice with 1x PBS also by centrifugation. The final solution was then stored at 4°C.

### Example 16

### Assay for HBV DNA Using HRP-DNA Probe and a Biotinylated Probe Bound to an Avidin Bead

Biotin labeled probe (B'; 1000 pmoles in 66.7 µl of water) was combined with 5 ml of a 0.25% (w/v) solution of 0.8 µ avidin beads (Pandex laboratories), 1 ml of 20x SSC, 0.5 ml of 1% NP40 and 0.6 ml of 1 mg/ml polyA. After 1 h at 37°C, the beads were washed twice by centrifugation with 4x SSC, 0.1% NP40 then stored in 2.5 ml of this solution. The HBV analyte (described above) in 3 µl water was diluted into 10 µl of 4x SSC, 1% SDS, 0.5 M NaOH and 1.5 pmoles of the labeling and capturing probe sets. The mixture was heated to 95°C for 10 min., cooled on ice and neutralized with 5 µl of 1 M acetic acid, then 10 µl of the biotin probe beads were added and the solution was incubated at 37°C for 1 h.

The beads were washed twice by centrifugation with 4X SSC, 0.1% NP40, then taken up in 50 µl of 0.1% NP40, 1 mg/ml polyA, 10 mg/ml BSA, 1X PBS containing 1 pmole of HRP-DNA conjugate and set a 37°C for 1 h. The beads were washed with 0.1% NP40, 1X PBS three times then transferred in 50 µl to a microtiter dish. To each well, 50 µl of fresh OPD solution (98 mg OPD (O-phenylenediamine), 20 µl of 30% H₂O₂ in 10 ml of 50 mM sodium citrate pH 5.0) was added, mixed and set 5 min. at 37°C. The absorbances were recorded on a microtiter plate reader. Control hybridizations contained no HBV analyte.

**Table 4**

| Condition | Absorbance Reading |
|---|---|
| 1 pmole | >2 |
| 0.1 pmole | >2 |
| 0.01 pmole | 0.88 ± 0.23 |
| 1 fmole | 0.20 ± 0.05 |
| 0.1 fmole | 0.07 ± 0.03 |
| NO ANALYTE | 0.01 ± 0.01 |

## Claims

1. A modified nucleotide given by the structure, 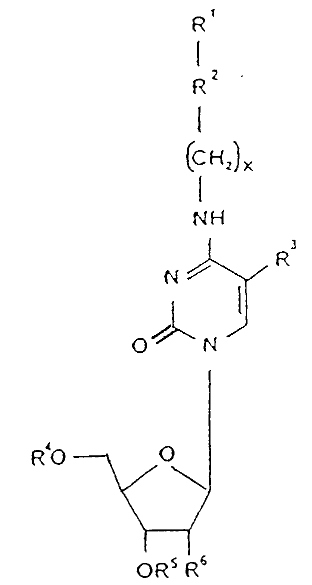 wherein R¹ is a reactive group derivatizable with a detectable label, R² is a linking moiety selected from: 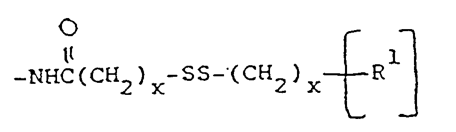 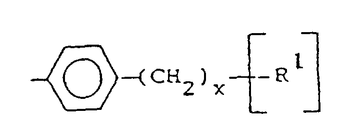 wherein x is an integer in the range of 1 to 8 inclusive, R³ is selected from the group consisting of hydrogen, methyl, bromine, fluorine and iodine, R⁴ is selected from the group consisting of hydrogen, an acid-sensitive, base-stable blocking group or an acyl capping group, R⁵ is hydrogen or a phosphorus derivative, R⁶ is H, OH, or OR where R is an acid-sensitive, base-stable protecting group, x is an integer in the range of 1 to 8 inclusive.

2. A method of making a modified nucleotide as claimed in claim 1 comprising the steps of:
reacting a pyrimidine nucleotide having the structure: 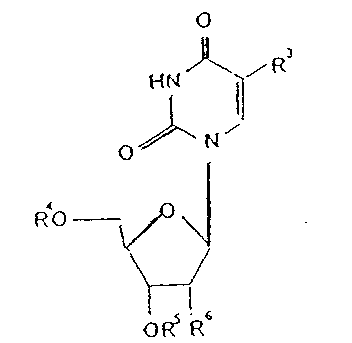 where said nucleotide is thymine, unsubstituted uracil, or uracil substituted at the 5-position with R³, wherein R³ is selected from the group consisting of hydrogen, methyl, fluorine, bromine and iodine, R⁴ and R⁵ are hydrogen, and R⁶ is hydrogen, hydroxyl, or blocked hydroxyl, with at least one protecting compound, thereby producing a 3' - and 5'- protected nucleotide: reacting said protected nucleotide with an activating agent, thereby providing an amine moiety on said nucleotide;
reacting the amine moiety of said nucleotide with a linking agent, thereby providing a structure having extending from the 4-position of said pyrimidine a linking moiety selected from 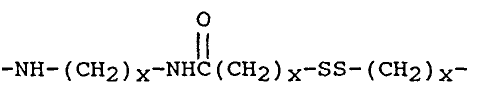 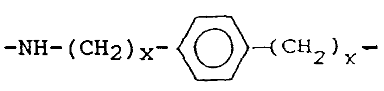 wherein X is an integer in the range of 1 to 8 inclusive and further having a free amine, carboxylic acid, or sulfhydryl reactive moiety;
reacting said free amine, carboxylic acid, or sulfhydryl moiety with a caproic acid reagent selected from the group consisting of caproic acid, an activated caproic acid ester, 6-aminocaproic acid and combinations thereof.

3. Polynucleotide probes having at least two nucleotides, at least one of which is given by the structure. 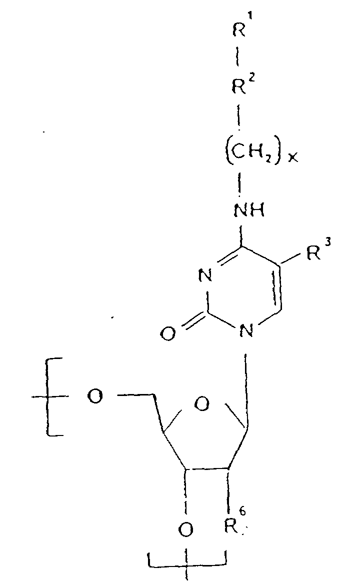 wherein R¹ is a reactive group derivatized with a detectable label, R² is a linking moiety selected from 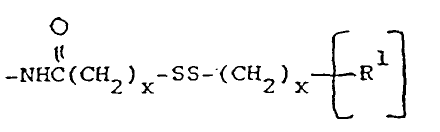 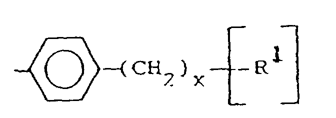 wherein x is an integer in the range of 1 to 8 inclusive, R³ is selected from the group consisting of hydrogen, methyl, bromine, fluorine and iodine, R⁶ is H, OH, or OR where R is a protecting group and x is an integer in the range of 1 to 8 inclusive.

4. A method of detecting a nucleotide sequence in a sample containing single-stranded or double-stranded DNA or RNA, comprising the steps of:
providing an analyte having a nucleotide sequence:
providing a labelled polynucleotide probe having a nucleotide sequence complementary to said analyte sequence, said polynucleotide probe including at least one nucleotide having the structure: 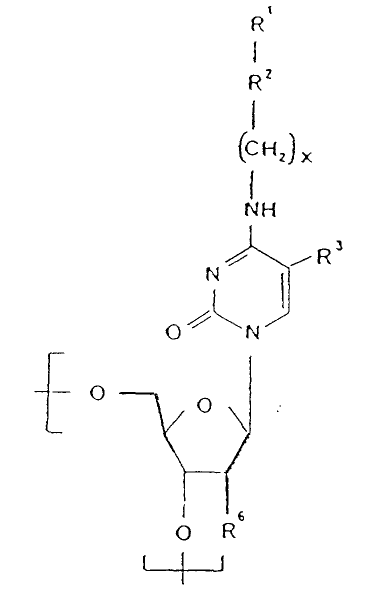 wherein R¹ is a reactive group derivatized with a detectable label, R² is a linking moiety selected from 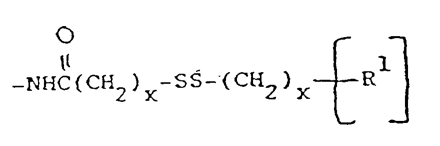 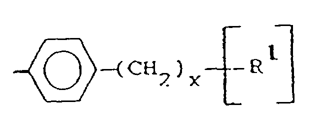 wherein x is an integer in the range of 1 to 8 inclusive, R³ is selected from the group consisting of hydrogen, methyl, bromine, fluorine and iodine, R⁶ is H, OH or OR where R is a protecting group and x is an integer in the range of 1 to 8 inclusive:
contacting said analyte with said labelled probe for a time sufficient for nucleic acid complexes to form: and
detecting the presence of any such nucleic acid complexes.

5. The method of claim 4 wherein said labelled polynucleotide probe is bound to a solid support.

## Patentansprüche

1. Ein modifiziertes Nucleotid der allgemeinen Formel 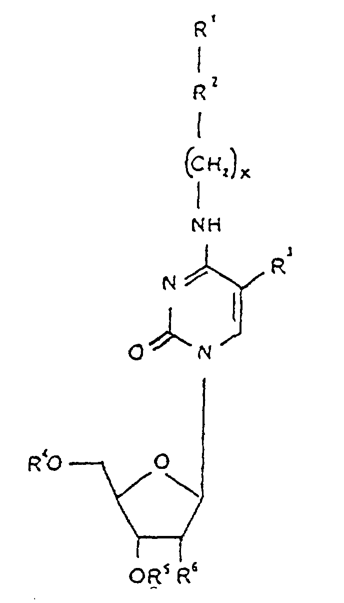 worin R¹ eine mit einem nachweisbaren Marker derivatisierbare reaktive Gruppe ist, R² ein verbindender Rest ausgewählt aus 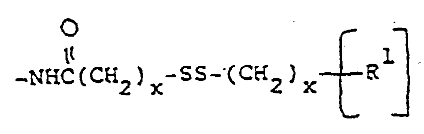 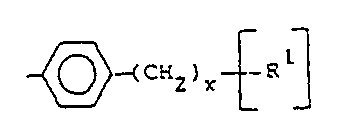 ist, wobei x eine ganze Zahl im Bereich von 1 bis einschließlich 8 ist, R³ aus der Gruppe bestehend aus einem Wasserstoffatom, einer Methylgruppe, einem Brom-, Fluor- und lodatom ausgewählt ist, R⁴ aus der Gruppe bestehend aus einem Wasserstoffatom, einer säureempfindlichen, basenstabilen Blockierungsgruppe oder einer Acyl-Schutzgruppe ausgewählt ist, R⁵ ein Wasserstoffatom oder ein Phosphorderivat ist, R⁶ ein Wasserstoffatom, die Gruppe OH oder OR ist, worin R eine säureempfindliche, basenstabile Schutzgruppe ist und x eine ganze Zahl im Bereich von 1 bis einschließlich 8 ist.

2. Verfahren zur Herstellung eines modifizierten Nucleotids gemäß Anspruch 1, umfassend die Schritte:
Umsetzen eines Pyrimidin-Nucleotids der allgemeinen Formel 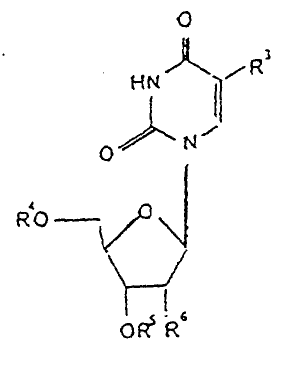 wobei das Nucleotid Thymin, unsubstituiertes Uracil oder Uracil ist, das an der 5-Position mit R³ substituiert ist, wobei R³ aus der Gruppe bestehend aus einem Wasserstoffatom, einer Methylgruppe, einem Fluor-, Brom- und Todatom ausgewählt ist, R⁴ und R⁵ Wasserstoffatome sind und R⁶ ein Wasserstoffatom, eine Hydroxylgruppe oder eine blockierte Hydroxylgruppe ist,
mit mindestens einer schützenden Verbindung, wodurch ein 3'- und 5'-geschütztes Nucleotid entsteht,
Umsetzen des geschützten Nucleotids mit einem Aktivierungsmittel, wodurch ein Aminrest in das Nucleotid eingeführt wird,
Umsetzen des Aminrests des Nucleotids mit einem verbindenden Mittel, wodurch eine Struktur erzeugt wird, die einen verbindenden Rest ausgewählt aus 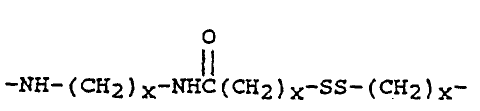 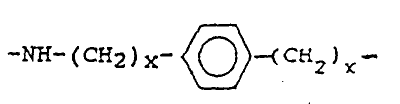 gebunden an der 4-Position des Pyrimidins aufweist, wobei x eine ganze Zahl im Bereich von 1 bis einschließlich 8 ist und wobei die Struktur weiterhin einen reaktiven Rest aufweist, der ein freier Amin-, Carbonsäure- oder Sulfhydrylrest ist, und
Umsetzen des freien Amin-, Carbonsäure- oder Sulfhydrylrests mit einem n-Capronsäurereagenz ausgewählt aus der Gruppe bestehend aus n-Capronsäure, einem aktivierten n-Capronsäureester, 6-Amino-n-capronsäure und Kombinationen davon.

3. Polynucleotidsonden mit mindestens zwei Nucleotiden, wobei mindestens eines davon die allgemeine Formel 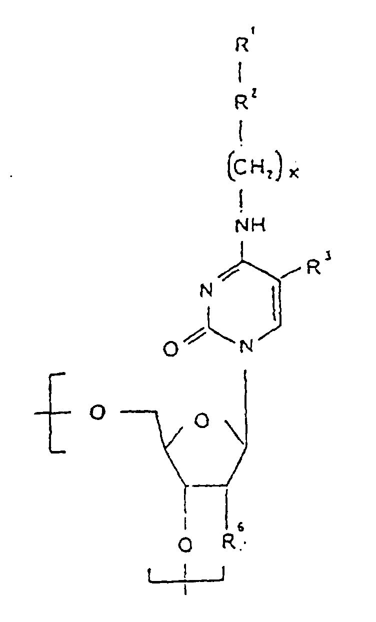 hat, worin R¹ eine mit einem nachweisbaren Marker derivatisierte reaktive Gruppe ist, R² ein verbindender Rest ausgewählt aus 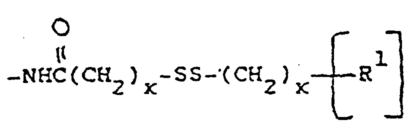 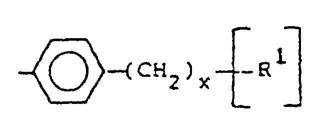 ist, wobei x eine ganze Zahl im Bereich von 1 bis einschließlich 8 ist, R³ aus der Gruppe bestehend aus einem Wasserstoffatom, einer Methylgruppe, einem Brom-, Fluor- und lodatom ausgewählt ist, R⁶ ein Wasserstoffatom, die Gruppe OH oder OR ist, worin R eine Schutzgruppe ist und x eine ganze Zahl im Bereich von 1 bis einschließlich 8 ist.

4. Verfahren zum Nachweis einer Nucleotidsequenz in einer Probe, die eine einzelsträngige oder doppelsträngige DNA oder RNA enthält, umfassend die Schritte:
Bereitstellen eines Analyten mit einer Nucleotidsequenz,
Bereitstellen einer markierten Polynucleotidsonde mit einer Nucleotidsequenz, die komplementär zu der Sequenz des Analyten ist, wobei die Polynucleotidsonde mindestens ein Nucleotid der allgemeinen Fermel 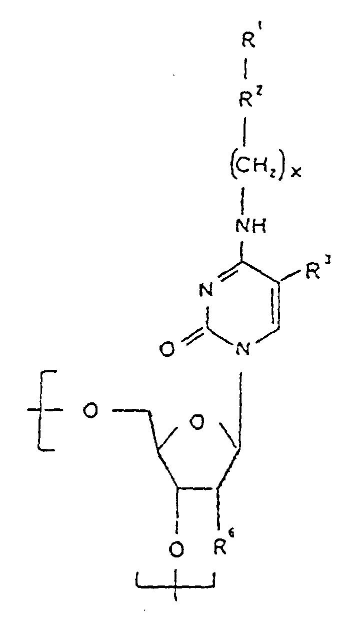 umfasst, worin R¹ eine mit einem nachweisbaren Marker derivatisierte reaktive Gruppe ist, R² ein verbindender Rest ausgewählt aus 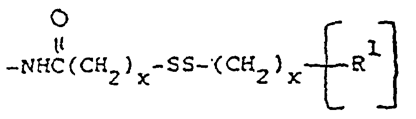 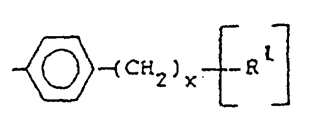 ist, wobei x eine ganze Zahl im Bereich von 1 bis einschließlich 8 ist, R³ aus der Gruppe bestehend aus einem Wasserstoffatom, einer Methylgruppe, einem Brom-, Fluor- und lodatom ausgewählt ist, R⁶ ein Wasserstoffatom, die Gruppe OH oder OR ist, worin R eine Schutzgruppe ist und x eine ganze Zahl im Bereich von 1 bis einschließlich 8 ist,
In-Kontakt-Bringen des Analyten mit der markierten Sonde für einen Zeitraum, der zur Bildung von Nucleinsäurekomplexen ausreichend ist, und
Nachweisen der Gegenwart eines solchen Nucleinsäurekomplexes.

5. Verfahren nach Anspruch 4, wobei die markierte Polynucleotidsonde an ein Trägermaterial gebunden ist.

## Revendications

1. Nucléotide modifié donné par la structure, 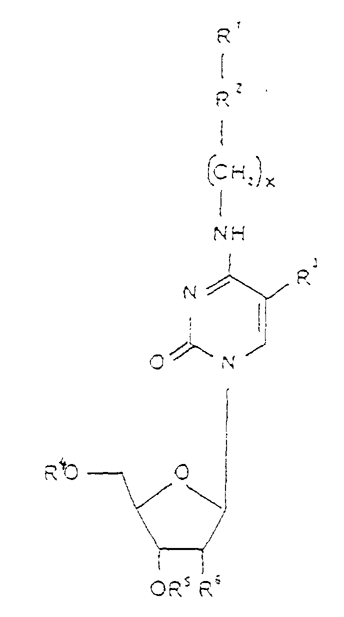 dans laquelle R¹ est un groupe réactif dérivable avec un marqueur détectable, R² est un résidu de liaison choisi parmi : 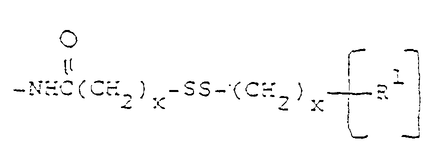 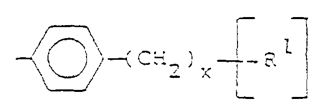 où x est un nombre entier compris de façon inclusive entre 1 et 8, R³ est choisi parmi le groupe consistant en hydrogène, méthyle, brome, fluor et iode, R⁴ est choisi parmi le groupe consistant en un hydrogène, un groupe bloquant sensible aux acides et stable en milieu basique ou un groupe acyle de coiffage, R⁵ est un hydrogène ou un dérivé de phosphore, R⁶ est H, OH ou OR où R est un groupe protecteur sensible aux acides et stable en milieu basique, x est un entier compris de façon inclusive entre 1 et 8.

2. Procédé de préparation d'un nucléotide modifié selon la revendication 1 comprenant les étapes consistant à :
faire réagir un nucléotide pyrimidinique ayant la structure: 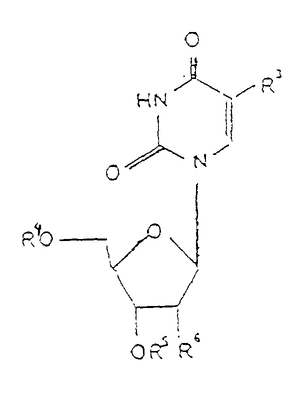 dans laquelle ledit nucléotide est une thymine, une uracile non substituée, ou une uracile substituée en position 5 par R³, R³ étant choisi dans le groupe consistant en hydrogène, méthyle, fluor, brome et iode, R⁴ et R⁵ étant de l'hydrogène et R⁶ étant de l'hydrogène, un hydroxyle ou un hydroxyle bloqué, avec au moins un composé protecteur, produisant ainsi un nucléotide protégé en 3' et 5';
faire réagir ledit nucléotide protégé avec un agent activant, fournissant ainsi un résidu amine sur ledit nucléotide;
faire réagir le résidu amine dudit nucléotide avec un agent de liaison, fournissant ainsi une structure ayant, s'étendant à partir de la position 4 de ladite pyrimidine, un résidu de liaison choisi parmi 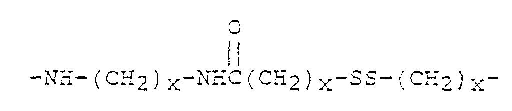 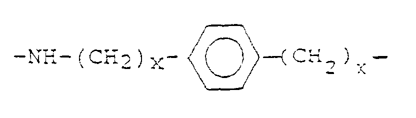 où x est un entier compris de façon inclusive entre 1 et 8 et comprenant en outre un résidu réactif d'amine libre, d'acide carboxylique ou de sulfhydryle;
faire réagir ledit résidu d'amine libre, acide carboxylique ou sulfhydryle avec un réactif de type acide caproïque choisi dans le groupe consistant en acide caproïque, ester de l'acide caproïque activé, acide 6-aminocaproïque et combinaisons de ceux-ci.

3. Sondes polynucléotidiques ayant aux moins deux nucléotides, au moins l'un d'entre eux étant donné par la structure 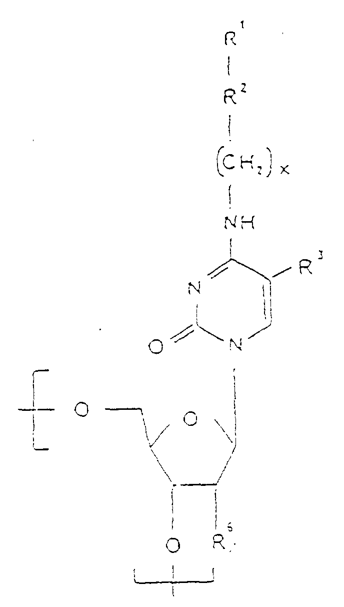 où R¹ est un groupe réactif dérivé avec un marqueur détectable, R² est un résidu de liaison choisi parmi 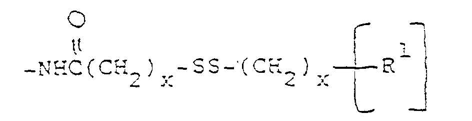 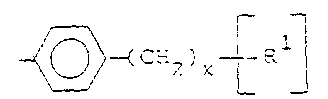 où x est un entier compris de façon inclusive entre 1 et 8, R³ est choisi dans le groupe consistant en hydrogène, méthyle, brome, fluor et iode, R⁶ est H, OH ou OR, où R est un groupe protecteur et x est un entier compris de façon inclusive entre 1 et 8.

4. Méthode de détection d'une séquence nucléotidique dans un échantillon contenant de l'ARN ou de l'ADN simple-brin ou double-brin, comprenant les étapes consistant à :
fournir un analyte ayant une séquence nucléotidique;
fournir un polynucléotidique marqué ayant une séquence nucléotidique complémentaire de ladite séquence de l'analyte, ladite sonde polynucléotidique incluant au moins un nucléotide ayant la structure: 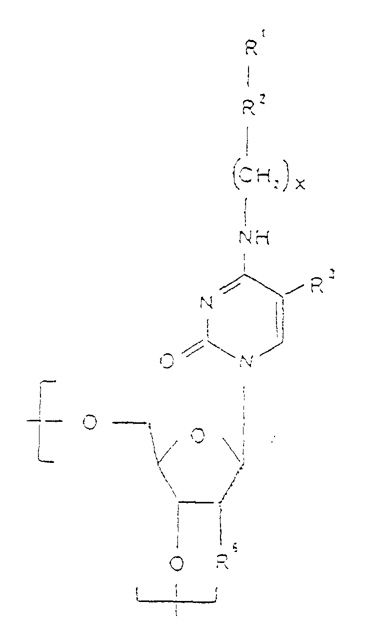 où R¹ est un groupe réactif dérivé avec un marqueur détectable, R² est un résidu de liaison choisi parmi 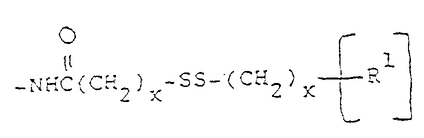 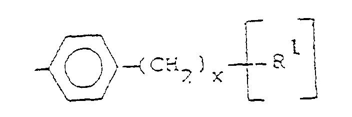 où x est un entier compris de façon inclusive entre 1 et 8, R³ est choisi dans le groupe consistant en hydrogène, méthyle, brome, fluor et iode, R⁶ est H, OH et OR, où R est un groupe protecteur et x est un entier compris de façon inclusive entre 1 et 8;
la mise en contact dudit analyte avec ladite sonde marquée pendant un temps suffisant pour que des complexes d'acide nucléique se forment; et
détecter la présence de tels complexes d'acide nucléique.

5. Méthode selon la revendication 4, dans laquelle ladite sonde polynucléotidique marquée est liée à un support solide.
